# EUROPEAN PATENT SPECIFICATION

(11) **EP 3 866 763 B1**
(45) Date of publication and mention of the grant of the patent: **24.07.2024**
(21) Application number: 19798479.2
(22) Date of filing: 18.10.2019
(51) Int. Cl.: A61K 47/02, A61K 47/32, A61K 9/06, A61K 9/08, A61L 27/52, A61L 27/26, A61L 27/38, B33Y 80/00

(54) **BIOINK FOR 3D DEPOSITION**
BIOTINTE ZUR DREIDIMENSIONALEN FORMUNG
BIO-ENCRE POUR UNE 3D DEPOSITION

(30) Priority: 18.10.2018 US 201862747490 P
(43) Date of publication of application: 25.08.2021
(73) Proprietor: Regents of the University of Minnesota, Minneapolis, MN 55455 (US)
(72) Inventor: OGLE, Brenda M., Edina, Minnesota 55439 (US); LIN, Wei-Han, Minneapolis, Minnesota 55414 (US); KUPFER, Molly E., Minneapolis, Minnesota 55410 (US); BHUIYAN, Didarul, Milwaukee, Wisconsin 53209 (US)
(74) Representative: Murgitroyd & Company
(86) International application number: PCT/US2019/057009
(87) International publication number: WO 2020/081982

(56) References cited:
- WO-A1-2017/214592
- WO-A1-2018/071639
- WO-A1-2018/078130
- YIN-J. YAN-M. WANG-Y. FU-J. SUO-H.: "3D Bioprinting of Low-Concentration Cell-Laden Gelatin Methacrylate (GelMA) Bioinks with a Two-Step Cross-linking Strategy.", ACS APPLIED MATERIALS AND INTERFACES, vol. 10, no. 8, 28 February 2018 (2018-02-28), pages 6849 - 6857, XP002797472

## Description

### GOVERNMENT INTEREST

This invention was made with government support under HL137204 awarded by the National Institutes of Health (NIH). The government has certain rights in the invention.

### TECHNICAL FIELD

This disclosure generally relates to bioink for three-dimensional printing of tissue structures.

### BACKGROUND

Bioprinting includes the application of three-dimensional printing techniques for deposition of biological materials into desired patterns. Cell patterns are created layer-by-layer, such that cell function and viability can be preserved in the resulting printed construct and can be used for medical and/or tissue engineering purposes. WO 2017/214592, WO 2018/071639 , WO 2018/078130, Yan-M. ACS APPLIED MATERIALS AND INTERFACES, vol. 10, no. 8, 28 February 2018 disclose related bioink compositions.

### SUMMARY

Bioinks that can be used for three-dimensional (3D) printing of structures, and associate printable structures, are described. In one example, a bioink composition may include gelatin methacrylate, collagen methacrylate, and lithium phenyl-2,4,6-trimethylbenzoylphosphinate (LAP) according to claim 5 although other formulations, such as different formulations including various contributions of gelatin methacrylate and collagen methacrylate, for a bioink are described as well. In some examples, the bioink may also include fibronectin and laminin. The bioink examples described herein may be configured to promote stem cell differentiation into specific cell types, such as cardiomyocytes, that can proliferate into functional 3D structures, such as fluid pumps, for example. In some examples, already differentiated cells may be applied using the bioink to proliferate into functional 3D structures.

The bioink composition includes gelatin methacrylate and collagen methacrylate according to claim 1.

In another example, a bioink composition includes gelatin methacrylate, collagen methacrylate, lithium phenyl-2,4,6-trimethylbenzoylphosphinate, fibronectin, laminin, and a solvent comprising mTeSR medium, acetic acid, and sodium hydroxide (NaOH).

In another example, a method includes printing a three-dimensional structure using a bioink comprised of gelatin methacrylate, collagen methacrylate, and lithium phenyl-2,4,6-trimethylbenzoylphosphinate.

The details of one or more examples of the techniques of this disclosure are set forth in the accompanying drawings and the description below. Other features, objects, and advantages of the techniques will be apparent from the description and drawings, and from the claims.

### BRIEF DESCRIPTION OF DRAWINGS

FIG. 1A is a conceptual diagram of a cross-sectional of an example human chambered cardiac pump (hChaMP).
FIG. 1B is a flow diagram of a method for creating the example hChaMP of FIG. 1A.
FIG. 2A includes images of example cell viability and proliferation using different examples of bioinks described herein.
FIG. 2B is a matrix illustrating a comparison between different examples of bioinks described herein.
FIG. 3 is a graph illustrating stiffness for an example printed bioink structure.
FIG. 4 includes images of an example 3D printing method using a bioink described herein.
FIGS. 5A, 5B, and 5C includes images and graphs representing the functionality of printed cardiac tissues using an example bioink described herein.
FIGS. 6A and 6B are a conceptual diagrams illustrating an example 3D printed heart model.
FIG. 7 includes images illustrating example stem cell colonies.
FIG. 8 includes images illustrating an example 3D printing method using an example bioink.
FIG. 9 is a graph of calcium transients of an example printed structure.
FIG. 10 is MRI scan data of a printed heart and heat map showing the different of a real model and a printed heart.
FIG. 11A is a graph of pressure dynamics of an example printed heart using bioink described herein.
FIG. 11B is an optical map of an example printed heart using bioink described herein.
FIG. 12 is a conceptual diagram of a technique to form example gels with a bioink.
FIG. 13 is a group of images of example cell area coverage for respective different bioink formulations.
FIG. 14A is an image of example cardiomyocyte differentiation at day 32 for an example bioink formulation.
FIG. 14B is a graph of storage modulus and loss modulus for an example bioink formulation.
FIG. 14C is a graph of example DNA content for differentiated cardiomyocytes from an example bioink compared to native cardiac tissue.
FIG. 15A is a cross-sectional view of a 3D model of an example intact human heart (Template).
FIG. 15B is a cross-sectional view of an example 3D printed structure using an example bioink formulation (Print).
FIG. 16 is heat map showing geometric differences between the Template and Print versions of FIGS. 15A and 15B.
FIG. 17 is an image of an example printed structure using a bioink described herein.
FIGS. 18A, 18B, 18C, and 18D are images of an example process of perfusion of fluid through a printed chambered structure.
FIG. 19 is a conceptual diagram of a technique to combine an example bioink with hiPSCs (human induced pluripotent stem cells) bioprinted to form an hChaMP.
FIGS. 20A and 20B are images of example colonies of hiPSCs at different days.
FIG. 20C is a graph of the percent of total area occupied by the colonies at day zero.
FIGS. 21A, 21B, and 21C are images of immunofluorescence of example colonies at six weeks following lactate treatment and Ki67 staining.
FIG. 21 D is a graph of the percent of total area occupied by the colonies in FIGS. 21A-21C.
FIGS. 22A, 22B, and 22C are images of immunofluorescence of example colonies at six weeks following lactate treatment and TUNEL staining.
FIG. 22D is a graph of the percent of total area occupied by the colonies in FIGS. 22A-22C.
FIGS. 23A, 23B, and 23C are images of immunofluorescence of example colonies at six weeks following lactate treatment and respective cTnl, αSMA, and CD31 staining.
FIG. 23D is a graph of the percent of total area occupied by the colonies in FIGS. 23A-23C.
FIGS. 24A, 24B, and 24C are brightfield images of an example hChaMP portions stained for cardiac troponin T (cTnT).
FIGS. 25A, 25B, and 25C are images of cardiomyocytes of example hChaMPs following differentiation and immunolabeling for a Cx43 marker.
FIG. 25D is a graph of the intensity of staining for the marker of FIGS. 25A-25C.
FIGS. 26A, 26B, and 26C are images of cardiomyocytes of example hChaMPs following differentiation and immunolabeling for a Kir2.1 marker.
FIG. 26D is a graph of the intensity of staining for the marker of FIGS. 26A-26C.
FIGS. 27A, 27B, and 27C are images of cardiomyocytes of example hChaMPs following differentiation and immunolabeling for a Bin1 marker.
FIG. 27D is a graph of the intensity of staining for the marker of FIGS. 27A-27C.
FIGS. 28A, 28B, and 28C are images of cardiomyocytes of example hChaMPs following differentiation and immunolabeling for a RyR2 marker.
FIG. 28D is a graph of the intensity of staining for the marker of FIGS. 28A-28C.
FIGS. 29A, 29B, and 29C are images of cardiomyocytes of example hChaMPs following differentiation and immunolabeling for a SERCA2 marker.
FIG. 29D is a graph of the intensity of staining for the marker of FIGS. 29A-29C.
FIGS. 30A and 30B are graphs of peak amplitude and interspike interval for calcium transient activity for hChaMPs over time following differentiation.
FIG. 31 is a graph of an example dose-response effect of isoproterenol in example hChaMPs.
FIG. 32 are time-sequential images of voltage propagation in an example hChaMP.
FIGS. 33A, 33B, and 33C are images of example spontaneous electrical activity, activation time, and action potential duration of a hChaMP.
FIG. 33D is a graph of the mean APD80 of example hChaMPs.
FIGS. 34A, 34B, 34C, and 34D are images of example voltage activity and electrical activity for different pacing, respectively.
FIGS. 35A and 35B are graphs of spontaneous or isoproterenol-induced activity of an example hChaMP.
FIG. 36 is a graph of the coverage area of an example hChaMPs with spontaneous activity.
FIGS. 37A and 37B are graphs of contractility in terms of beats per minute and contractility rate, respectively, for example cardiomyocytes of hChaMPs.
FIG. 38 is a graph of an example dose-response effect of isoproterenol in terms of beat rate in example hChaMPs.
FIG. 39 is conceptual diagraph of an example system for measuring interchamber pressure and volume of a hChaMP.
FIG. 40 is a graph of intra-chamber pressure for an example hChaMP during ventricle beating.
FIG. 41 is a graph of beat rate for an example hChaMP during ventricle beating.
FIGS. 42A and 42B are graphs of intra-chamber pressure and volume dynamics for example hChaMPs without and with isoproterenol.
FIG. 43 is a graph of an example isoproterenol response in terms of beat rate for example hChaMPs.
FIGS. 44A and 44B are graphs of volume and pressure with and without isoproterenol for an example hChaMP.
FIG. 45 is a flow diagram of an example 3D printing method using a bioink composition described herein.

### DETAILED DESCRIPTION

The disclosure describes compositions for a bioink that can be used for three-dimensional (3D) printing of structures. A goal of cardiac tissue engineering is to generate in vitro model systems that aid in our understanding of physiological and anatomy. For example, it may be desirable to further understanding of the heart with respect to development, growth and disease in order to create therapeutics that alleviate symptoms and improve survival outcomes. The field has effectively utilized pluripotent stem cells to derive all cardiac cell types with high efficiency and moved to the development of model systems often termed "microtissues" that take the form of microscale strips of tissue typically supported by flexible posts to mimic human muscle. These strips of tissue have shown great promise for testing of drugs with associated benefit to the pharmaceutical industry, but the strips of tissue also lack the capacity to accurately reproduce the architecture and functional output of structures such as heart chambers. This is a significant limitation because tissue performance cannot be directly compared with animal or human heart performance, which may be evaluated by measuring changes in chamber pressure and volume. In addition, cardiac remodeling with disease is substantially influenced by the complex flow profiles and associated force stimulation and response of the cardiac chambers.

A critical challenge for 3D bioprinting of complex tissue mimics is to formulate a bioink that supports printability and also cell health and function. Support of stem cell health and function may be particularly problematic, and bioinks that allow differentiation of specialized cell types are rare or nonexistent. For example, no known bioink has been created that supports the differentiation of cardiac muscle (i.e., cardiomyocytes). 3D bioprinting of specialized cell types may not be possible without bioinks that support and/or promote the differentiation of stem cells (e.g., induced pluripotent stem cells) after depositing (e.g., printing or otherwise disposing of the ink) into a desired structure.

Compositions of example bioinks are described herein that can be effectively deposited (e.g., printed) and support stem cell health and differentiation to specialized cell types such as cardiomyocytes. In other examples, the described bioinks may also support differentiation of deposited stem cells into many other cell types of soft tissues. These bioinks described herein may provide a material that can be printed and supports desired stem cell behavior, such as differentiation to cardiac muscle cells. The bioinks may, in some examples, also facilitate the production of cardiac tissue mimics for in vitro study of cell behavior, testing of cardiovascular devices and drugs, or implemented as a tissue-replacement therapeutic.

The bioink includes gelatin methacrylate and collagen methacrylate. In some examples, the bioink may also include lithium phenyl-2,4,6-trimethylbenzoylphosphinate (LAP). LAP may be a photoactivatable linker that may operate to link proteins, but other compounds or chemistries may be used instead of LAP in other examples. In some examples, the bioink may also include at least one of fibronectin or laminin. The bioink may include stem cells such as animal induced pluripotent stem cells or human induced pluripotent stem cells (hiPSCs), but other types of stem cells may be used in other examples. These stem cells may include cardiomyocyte precursors. In some examples, the bioink composition is configured to promote differentiation of human induced pluripotent stem cells into cardiomyocytes. In some examples, cardiomyocytes themselves may be included in a bioink formulation described herein. Although cardiac tissue and cardiomyocytes are described in exampled herein, the bioinks described herein may be applied to any cell type, such as cell types that may be challenging to manipulate to make engineered structures outside of the body. These other types of cells may not easily proliferate or migrate, but the bioink formulations described herein may facilitate proliferation and/or migration for any cell type. In addition, the bioink formulation, as applied any cell type, may be employed to print or otherwise create any type of one dimensional (a line of cells), two dimensional (a layer of cells), or three dimensional (several layers or a mold of cells) structure.

Several example formulations for a bioink are described herein. A bioink in accordance with the present invention includes between and including approximately 5-20 percent weight by volume of gelatin methacrylate and between and including approximately 0.1-1.0 percent weight by volume of collagen methacrylate. In an example, a bioink may include between and including approximately 8-12 percent weight by volume of gelatin methacrylate and between and including approximately 0.15-0.30 percent weight by volume of collagen methacrylate. In some examples, a bioink may include between and including approximately 0.1-2.0 percent weight by volume of lithium phenyl-2,4,6-trimethylbenzoylphosphinate. In some examples, a bioink may include between and including approximately 10-1000 micrograms per milliliter (µg/mL) of fibronectin. In some examples, a bioink may include between and including approximately 10-1000 µg/mL of laminin.

In one example, the bioink may include approximately 10 percent weight by volume of gelatin methacrylate, approximately 0.25 percent weight by volume of collagen methacrylate, and approximately 0.5 percent weight by volume of the lithium phenyl-2,4,6-trimethylbenzoylphosphinate. In another example, the bioink composition may include approximately 100 milligrams per milliliter (mg/mL) of collagen methacrylate, approximately 2.5 mg/mL of gelatin methacrylate, approximately 5 mg/mL of the of lithium phenyl-2,4,6-trimethylbenzoylphosphinate, approximately 93.8 µg/mL of fibronectin, and approximately 93.8 µg/mL of laminin.

In some examples, the bioink may include a solvent that includes a medium such as mTeSR medium, acetic acid, and/or sodium hydroxide (NaOH). In some examples, the bioink may include a solvent which includes between and including approximately 50-90 percent weight by volume of mTeSR1 medium, between and including approximately 10-50 percent weight by volume of 20mM acetic acid, and between and including approximately 0.5-2 percent weight by volume of 1M NaOH. In one example, the bioink may include the solvent which includes approximately 74 percent weight by volume of mTeSR medium, 20 percent weight by volume of 20mM acetic acid, and 1 percent weight by volume of 1M NaOH. In another example, a bioink composition may include gelatin methacrylate, collagen methacrylate, lithium phenyl-2,4,6-trimethylbenzoylphosphinate, fibronectin, laminin, and a solvent comprising mTeSR medium, acetic acid, and sodium hydroxide (NaOH).

The various examples of bioink described herein may be configured to enable 3D bioprinting of functional, complex cardiac structures. The bioink composition may leverage the interaction between extracellular matrix proteins and stem cells to enable differentiation of stem cells to specific cell types. Various combinations of extracellular matrix proteins supportive of the specification of individual cardiac cell types are described. In one example, a specific formulation can be used for cardiomyocyte specification to produce a bioink configured for the expansion of entrapped human pluripotent stem cells to densities approximating that of native tissue. This step facilitates deposition into structure because direct 3D printing of cardiomyocytes typically results in inadequate cell density since this cell type is capable of only modest proliferation and migration. The bioink may also promotes differentiation of stem cells to cardiomyocytes with high efficiency in a chambered heart structure because nutrient access can be a challenge with complex architecture and to date has limited complete differentiation throughout structures of this type. The bioink also promotes ease of printing down to approximately 250 micron minimum feature size, which provides the advantage of relatively low viscosity bioink of the type needed to support stem cell expansion while enabling high resolution printing. In addition, the bioinks described herein can be used to form of a structure on the scale of centimeters with enclosed chambers fed by vessel-shaped fluid inlet and outlet. After extended culture and associated cell expansion and differentiation after deposition of the bioink, the structure can be transformed from initially soft and fragile to a structure that can support fluid flow without leakage.

These bioinks described herein can provide utility in many fields, such as the field of cardiology with a structure that can provides access to a human model system that can sustain flow profiles and exhibit pressure-volume dynamics characteristic of the native heart. An example model of such a heart can therefore be useful for understanding remodeling associated with cardiac disease progression imposed by mechanical insult, genetic predisposition or diet, as some examples. These bioink printed structures can also be useful for testing drug toxicity or efficacy and, given the scale, be amenable to the testing of medical devices, implantation to the heterotopic position in mice, and may even be appropriate for clinical transplantation.

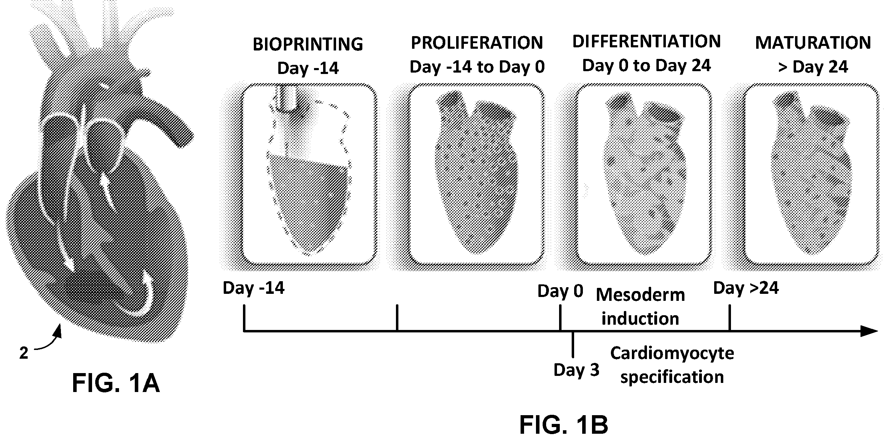

FIG. 1A is a conceptual diagram of a cross-sectional of an example human chambered cardiac pump (hChaMP), which is an example structure that may be created using the techniques described herein. As discussed herein, human cardiac muscle derived *ex vivo* may be in high demand as a substrate for testing drug efficacy and toxicity, as an analog for studying tissue remodeling associated with cardiac muscle damage or disease, and as a prelude to clinical cardiac repair. These tissue models may be created by harnessing the power of stem cell biology, biomaterials, and 3D fabrication technologies. Some engineered heart tissues consist of geometrically simple structures (strips or rings) made by casting cardiomyocytes in an extracellular matrix-based gel. These types of tissues can be attached to rigid or flexible posts in order to provide resistance against which the tissue can contract and to modulate mechanical loading. However, their lack of structural complexity limits their applicability for *in vitro* modeling of cardiac function and disease. Other volume-handling cardiac tissue models may be capable of recapitulating pressure-volume dynamics of the heart. However, these tissues are limited to a single ventricle model and thus lack the capacity for perfusion.

3D bioprinting may be a means to generate more complex tissues from the bottom-up. The ability to print tissues composed entirely of native proteins, cells and/or biocompatible synthetic components is possible and accessible. It is possible to print entire heart organ models using biological materials, but the resulting constructs may lacked cells or evidence of electromechanical function. The fact that macroscale contractile function has not yet been achieved in a 3D printed, chambered heart model is likely related to the challenges associated with handling mature cardiac muscle cells. More specifically, cardiomyocytes may not easily proliferate or migrate to populate vacant spaces in the 3D printed tissue mass, thus preventing the formation of cell-cell junctions throughout the construct. An alternative approach is to print stem cells, which are highly proliferative, and then induce maturation *in situ* following cell expansion.

To support proliferation, the bioink may be porous and/or susceptible to degradation such that stem cell colonies can expand unfettered. To support differentiation, the bioink may benefit from containing cell engagement motifs that enhance signaling associated with cardiomyocyte-specific differentiation. As described herein, stem cell maturation and differentiation and the proper function of cells derived thereof may be dependent on the temporal and spatial engagement of extracellular matrices (ECM) both in a given organ system during development and in the context of *ex vivo* stem cell culture. As one of many examples, mesoderm specification has been linked to α5β1 integrin activation. Engagement of this integrin by ECM (especially laminin511/111 and fibronectin) modulates BMP4 expression, which together with Wnt, fibroblast growth factor, and transforming growth factor-β/nodal/activin signaling, can mediate differentiation to mesoderm. In addition, engagement of fibroblast-derived ECM via β1, α2, and α3 integrins in human embryonic stem cells activates the Wnt/β-catenin pathway via the MEK-ERK pathway, which drives endoderm differentiation. Finally, fibronectin/integrin β1/ β-catenin signaling may promote the emergence of mesoderm from induced pluripotent stem cells. As described herein, there is a direct link between elements of the focal adhesion, namely integrin-linked kinase (II,K), with GSK3β, the primary antagonist of β-catenin.

The techniques and compositions described herein build on understanding of ECM engagement and stem cell differentiation by tapping ECM formulation to promote cardiomyocyte differentiation and incorporate this formulation into a bioink that is conducive to human induced pluripotent stem cell (hiPSC) proliferation and can be deposited with spatial fidelity. As described as one example herein, the end result is a living pump that mimics the chambers, wall structure, and large vessel conduits of a native heart while housing viable, densely packed and functional cardiomyocytes as shown in FIG. 1A. These example human chambered muscle pumps (hChaMPs) can be maintained long term and may pave the way for generating increasingly complex structures that could include spatially designated pacemaker cells and an associated conduction system, an epicardium to support remodeling with health, injury, or disease, as well as a host of other important attributes including an arterial and venous nutrient exchange system. In other examples, such bioinks and techniques described herein may be employed to create other circulatory structures or other living tissue structures.

Thus, as shown in FIG. 1A, the cross-sectional view of the design template 2 illustrates a chambered heart that can be produced using the bioink and techniques described herein. Template 2 was derived from an MRI scan of the human heart that was reduced in scale 10 times (1.3 cm at its longest axis, akin to the size of a murine heart) and modified to harbor a one way flow loop through the chambers of the heart template. In this manner, the template 2 can include a structurally complex cardiac tissue with contiguous muscle and associated pump function.

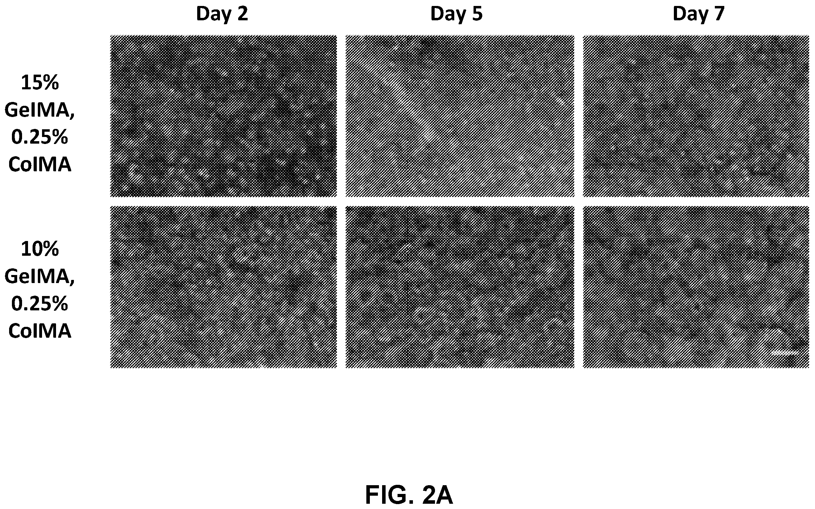

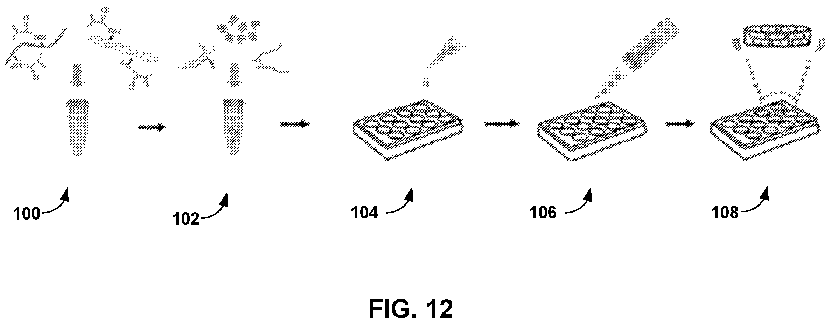

FIG. 1B is a flow diagram of a method for creating the example hChaMP of FIG. 1A. As shown in FIG. 1B, an example technique for creating a structure such as a hChaMP includes bioprinting, proliferation of cells (e.g., stem cells), differentiation which may include mesoderm induction and cardiomyocyte specification, and maturation of the cardiomyocytes. The bioprinting and proliferation process may take several days or weeks, such as 14 days. Maturation may occur after several more days or weeks after differentiation of the stem cells, such as more 24 or more days later. Cardiomyocyte specification may occur on day three, for example, after mesoderm induction. This organoid-like approach can be used to generate the hChaMP wherein human induced pluripotent cells (hiPSCs) are deposited with an optimized, ECM-based bioink that allowed for expansion of the hiPSCs to attain tissue-like densities and subsequent differentiation to cardiomyocytes. Over time, the hChaMP could beat synchronously, build pressure and move fluid akin to a living pump. This is one example technique to create such a structure. Such example techniques for generating a 3D structure using example bioinks are described below. One example experiment is discussed with respect to FIGS. 2A-11. Another example experiment is described with respect to FIGS. 12-44B. In any of these examples, different forumulations of an example bioink may be used to print, deposit, mold, or otherwise position cells (such as stem cells) into a desired structure for a specific functional purpose. For example, cardiomyocytes of a created hChaMP may contract to pump fluid through the hChaMP.

FIG. 2A includes images of example cell viability and proliferation using different examples of bioinks described herein. As described herein, 3D bioprinting of complex cardiac tissues utilizes a bioink that allows for expansion of cardiomyocyte precursors and then coupling (e.g., electromechanical coupling) of maturing cardiomyocytes. In the absence of these two critical factors (e.g., expansion of cardiomyocyte precurors and coupling of maturing cardiomyocytes) the printed structure may not attain cell densities that approach native tissue and therefore the potential for proper function. The example bioinks described herein are capable of these critical functions and demonstrate the function of complex cardiac tissue structures (beyond woodpiles, discs and rings) that finally capitalize on the power of 3D printing technology.

One example bioink includes 2.5 mg/mL collagen methacrylate (ColMA), 100 mg/mL gelatin methacrylate (GelMA), 93.8 µg/mL fibronectin (FN), 93.8 µg/mL laminin-III (LN), and 5 mg/mL lithium phenyl-2,4,6-trimethylbenzoylphosphinate (LAP). This low viscosity bioink may include pluripotent stem cells and can be printed into simple or complex structures using stereolithography or freeform reversible embedding of suspended hydrogels. Cells of structures printed in this manner can survive long term and proliferate at rates comparable to two-dimensional (2D) culture. Once a high cell density is attained, soluble factors to induce cardiomyocyte differentiation can be added to the culture media. Differentiation of functional cardiomyocytes to populate centimeter-scale, complex structures has been attained in this way and represents a step toward generating macrotissues capable of replicating pressure/volume relationships useful to the study of heart function with health and disease. In some examples, macrotissues of this type might also serve as a testbed for cardiac medical devices and/or therapeutic tissue grafting.

Replacement cardiac muscle is in great demand to treat a number of conditions including congenital heart defects, myocardial infarction and myocarditis. Combined, these conditions represent more than one-third of deaths in the US per year. 3D printing of scaffold materials and cells that reflect the composition of native tissue has been proposed as a means to generate replacement muscle. The concept is gaining traction, as the ability to print structures composed entirely of native protein, cells and/or synthetic components is possible and accessible to many laboratories. However, there are also very substantial hurdles, especially with respect to the inclusion of cells. In particular, inclusion of mature cardiac muscle cells is challenging as cardiomyocytes cannot easily proliferate or migrate to populate the 3D printed tissue mass with spatial acuity (i.e., the correct cell types in the correct location). If stem cells, which are highly proliferative, are used instead, cues for differentiation would be required and those cues differ between cardiac cell types.

Appropriate cues from the extracellular matrix may be used to overcome these hurdles. Stem cell maturation and differentiation and their respective normal function are dependent on the temporal and spatial specification of extracellular matrices (ECM) in a given organ system during developmental and with ex vivo stem cell culture. As one of many examples, mesoderm specification has been linked to α5β1 integrin activation. Engagement of this integrin by ECM (especially laminin511/111 and fibronectin) may modulate BMP4 expression, which together with Wnt, fibroblast growth factor, and transforming growth factor-β/nodal/activin signaling, can mediates this differentiation. Peptide activation of this integrin can also drive osteogenic differentiation of mesenchymal stem cells via the Wnt/β-catenin pathway activated via PI3K/Akt signaling. In addition, engagement of fibroblast-derived ECM via β1, α2, and α3 integrins in human embryonic stem cells can activate the Wnt/β-catenin pathway via the MEK-ERK pathway, which drives endoderm differentiation9. Finally, fibronectin/integrin β1/β-catenin signaling can promote the emergence of mesoderm from induced pluripotent stem cells. In this manner, there is a direct link between elements of the focal adhesion, namely integrin-linked kinase (ILK), with GSK3β, the primary antagonist of β-catenin.

As described further herein, ECM engagement and stem cell differentiation can be improved using a ECM formulation that promotes cardiomyocyte differentiation and fold this formulation into a bioink that can be deposited with spatial fidelity. The end result, in cardiac tissue examples, is a tissue that mimics the chambers and wall structure of a native heart, while housing living, viable, densely packed and functional cardiomyocytes. Structures of this type can be maintained long term and will pave the way for generating increasingly complex structures that could include specifically designated pacemaker cells and an associated conduction system, along with a host of other important attributes including an arterial and venous circulation.

As shown in FIG. 2A pluripotent cell viability and proliferation over the course of seven days are shown for two different examples of bioinks. The bioink at the bottom having approximately 10 percent weight by volume of gelatin methacrylate (GelMA) and approximately 0.25 percent weight by volume of collagen methacrylate (ColMA) indicates improved cell viability and differentiation as compared to the bioink at the top which includes approximately 15 percent weight by volume of GelMA and approximately 0.25 percent weight by volume of ColMA.

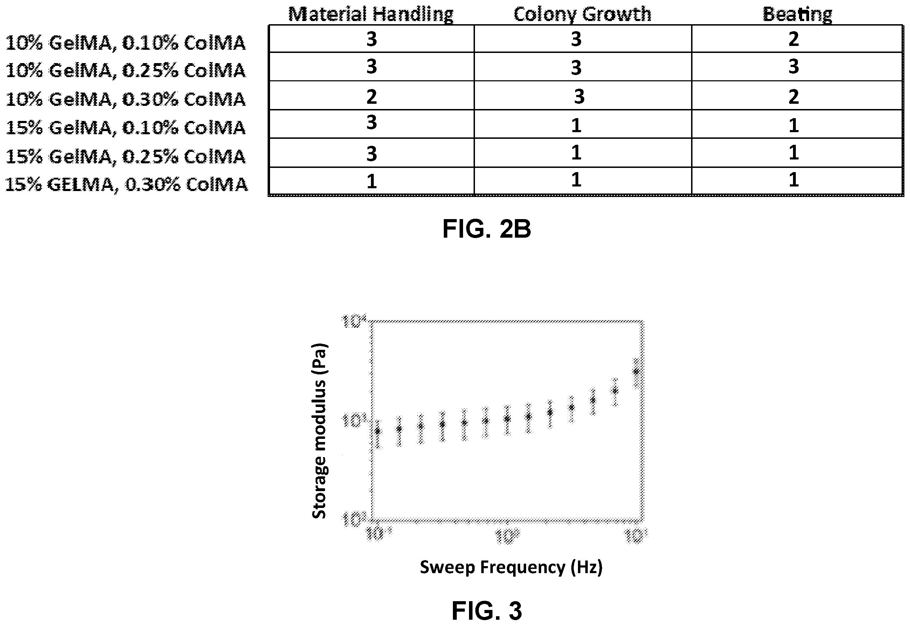

FIG. 2B is a matrix illustrating a comparison between different examples of bioinks described herein for material handling, colony growth, and beating or contraction of cardiomyocytes, according to one experiment. Higher numbers indicate improved performance for each category (e.g., a "3" indicates better performance than a "2," and a "2" indicates better performance than a "1"). According to this data, the bioinks containing 10% GelMA performed better than the bioinks containing 15% GelMA, with the bioink containing 10% GelMA and 0.25% ColMA performing the best of these six example formulations.

FIG. 3 is a graph illustrating stiffness for an example printed bioink structure. This mechanical characterizations of the stiffness (storage modulus) of the printed structure are similar to that of fetal heart tissue. As shown in FIG. 3, higher frequencies of sweep frequency resulted in increasing storage modulus of the bioink material. It is noted that higher concentrations of GelMA generally increase the viscosity of the formulation as well, and higher concentrations of GelMA may begin to degrade printability due to the higher viscosities.

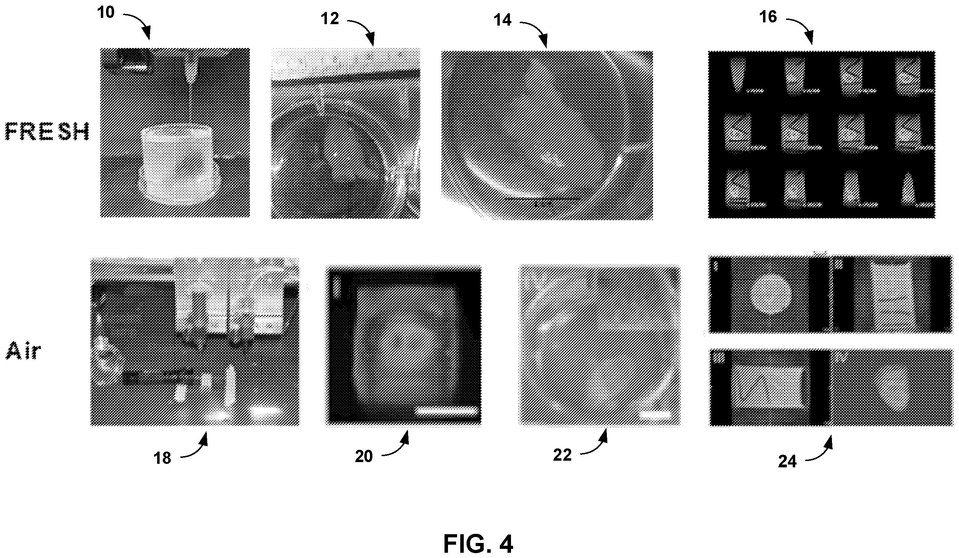

FIG. 4 includes images of an example 3D printing method using a bioink described herein. Bioprinting of ink may be optimized for pluripotent cell proliferation. Success was experienced with the FRESH method (printing into a gelatin slurry, TOP). The left top image 10 shows the actual print process, the middle shows post print structures 12 and 14, and the right shows an MRI scan 16 to confirm patent chamber openings and also to measure fidelity to the digital template. Bottom images of FIG. 4 show that extrusion in air with pluronic sacrificial material to buttress the chambers while printing is possible. The same panel series is shown for Air as for FRESH, such that image 18 indicates the print process, print structures 20 and 22 indicate the resulting structures, and MRI scan 24 is an example imaging of the print structures. The bioink described herein may be deposited using any printing or microfabrication method. Although the FRESH technique is described herein and may be advantageous when compared to the AIR method, the bioink formulations described herein may be used in any type of printing or microfabrication method. The methods described herein are just one example of such a method for implementing the described bioink examples.

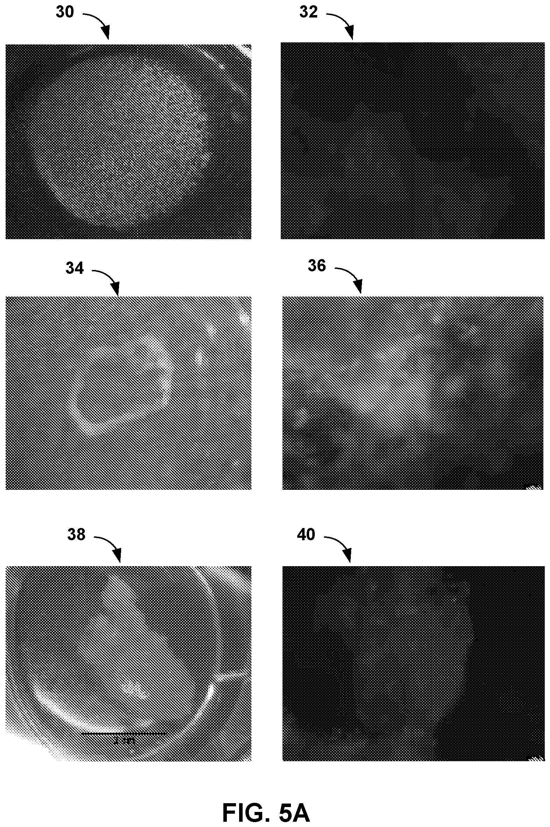

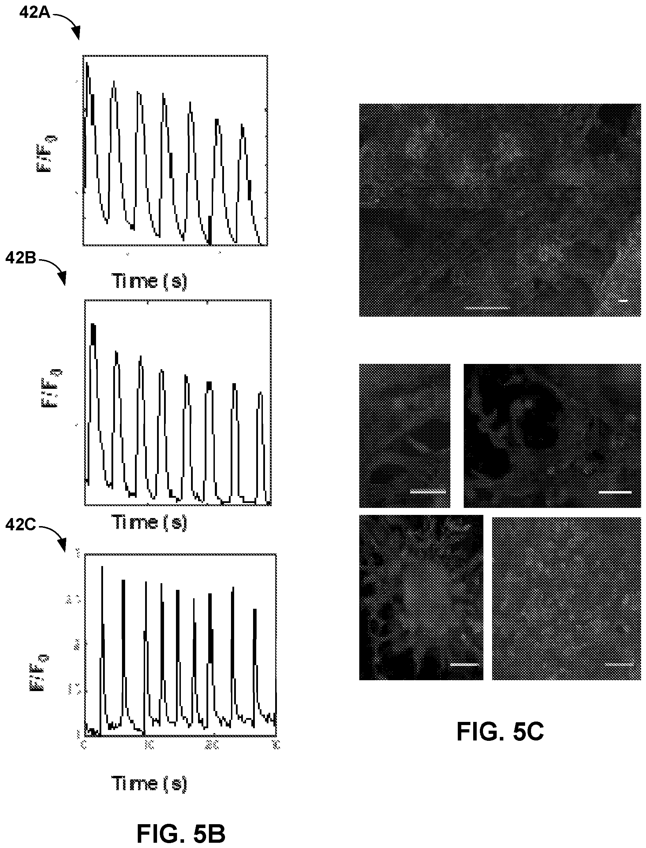

FIGS. 5A, 5B, and 5C includes images and graphs representing the functionality of printed cardiac tissues using an example bioink described herein. In addition to the components of the bioink that allow for polymerization after printing (e.g., ColMA, GelMA, LAP), additional extracellular matrix proteins may support and enhance cardiomyocyte differentiation, such as fibronectin and laminin. The combination of the first optimization (polymerization to promote proliferation) with the second optimization (to enable differentiation) may be used together for an example bioink. FIG. 5 shows printing of discs in images 30 and 32, rings in images 34 and 36, and a complex heart in images 38 an 40. FIG. 5B indicates representative traces 42A, 42B, and 42C of the respective disks (images 38 and 40), rings (images 34 and 36), and complex heart (images 38 and 40). FIG. 5C illustrates images corresponding to videos taken showing calcium transients throughout the tissue indicative of function of cardiomyocytes.

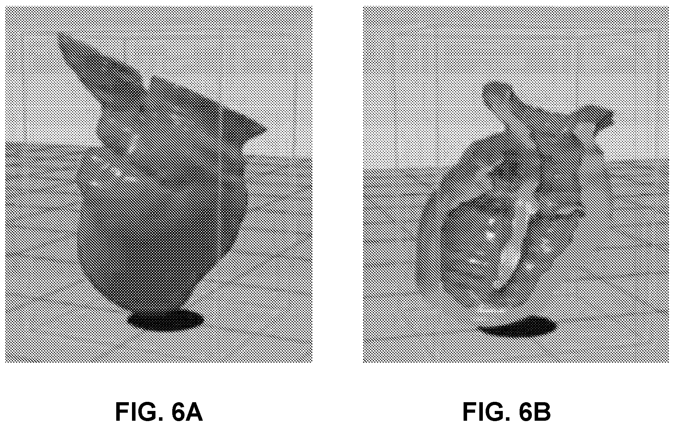

FIGS. 6A and 6B are conceptual diagrams illustrating an example 3D printed heart model. FIG. 6A is a perspective view of a 3D printed heart and FIF. 6B is a cross-sectional view of the 3D printed heart showing the chambers. For example, a 3D printed heart (e.g., a hChaMP) may include one or more chambers configured to draw fluid into an inlet of the heart and expel the fluid through an outlet of the heart. Contraction of the cardiac tissue of the printed heart may cause the chamber(s) to generate increased pressure within the chamber(s) to cause the fluid to flow out of the chamber and draw new fluid back into the chamber upon relaxation of the cardiac tissue. This operation may be similar to that of a mammalian heart, such as a human heart.

The bioink examples described herein may be used to create a structure that provides the capacity to shuttle ions within and between cells, contract in response to electrical gradients, and generate force all in the context of a complex cardiac tissue structure. For example, the bioink composition may include gelatin methacrylate (GelMA), collagen methacrylate (ColMA), fibronectin (FN), and laminin (LN) to support human-induced pluripotent stem cell (hiPSC) growth and differentiation, and LAP to photo-crosslink methacrylated components. A variety of ink compositions are described which can be used to print with hiPSCs followed by cardiomyocyte differentiation, which can be induced by modulating the Wnt/β-catenin pathway with small molecules. The example of FIGS. 6A and 6B is a printed a murine-scale human heart with a deviated septum to enable medium circulation in a bioreactor. Bioink containing hiPSCs was deposited using the FRESH printing method to accommodate the low-viscosity bioink. Briefly, bioink was extruded into a support bath composed of thermo-reversible gelatin micro-gel, which can be removed by raising temperature. The printing fidelity of the bioink can be verified by MRI scanning and CloudCompare. Imaging techniques can be used to verify cardiomyocyte phenotype, measure calcium transients, contractility, and voltage propagation.

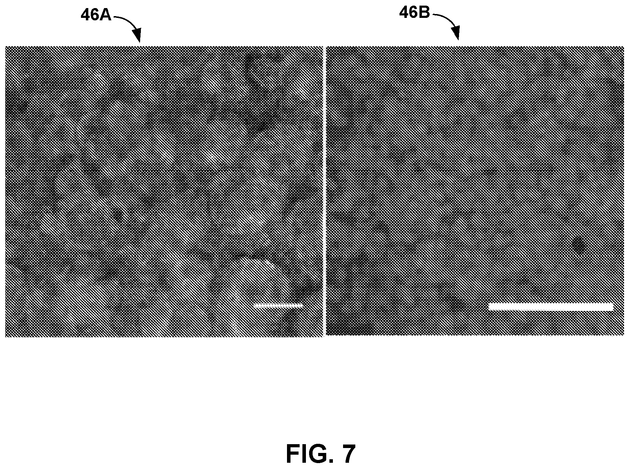

FIG. 7 includes images illustrating example stem cell colonies. As shown in FIG. 7, image 46A illustrates a bioink example supported formation of large hiPSC colonies crosslinked in the bioink over 7 days after gel formation. The ink also allowed cardiomyocyte differentiation, which was verified by immunostaining for cardiac troponin T (cTnT) (red) with nuclei counterstained with DAPI (blue), as shown in the image 46B of FIG. 7 (scale bar represents 100 µm).

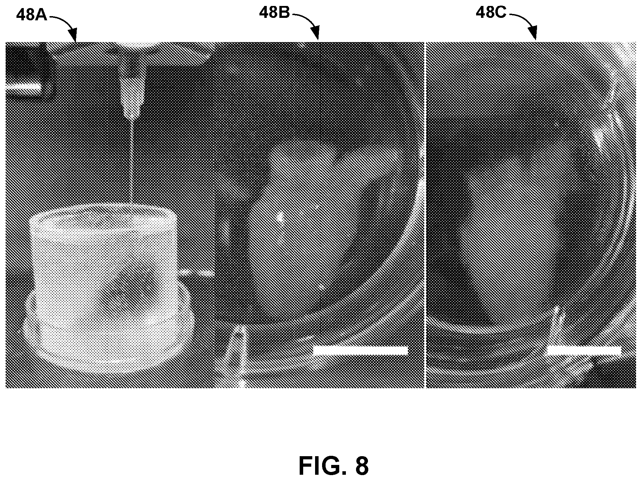

FIG. 8 includes images illustrating an example 3D printing method using an example bioink. Image 48A illustrates a clear upside down contour of the printed heart in the support bath could be discerned with FRESH printing with red food dye. After removing the support bath, the heart without cells (i.e., the heart in image 48B) and with cells (i.e., the heart in image 48C) both possessed an intact structure with clear vessels, while the heart without cells seemed to have a smoother surface. Scale bar in each of images 48B and 48C represents 1 cm.

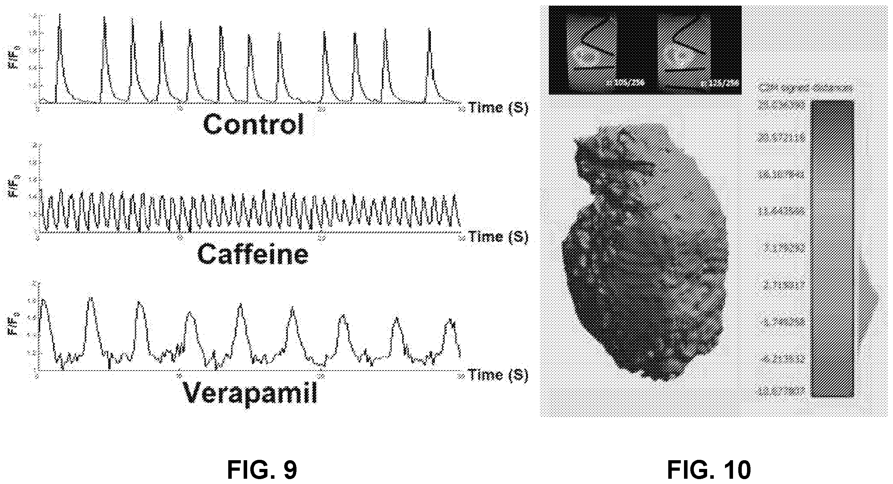

As shown in FIG. 9, calcium handling and ionotropic responses of the differentiated hiPSCs in the printed heart were confirmed by calcium traces. For the complex heart, FIG. 9 shows the response to drugs verapamil and caffeine to slow and accelerate the beating rate, respectively, with respect to the control beating rate. FIG. 10 is an MRI scan data of a printed heart and heat map showing the different of a real model and a printed heart. The MRI scan of the printed structure in FIG. 10 showed clear chamber and vessel structures inside the construct, which suggested the interconnectivity of the printed hearts and high fidelity to the template.

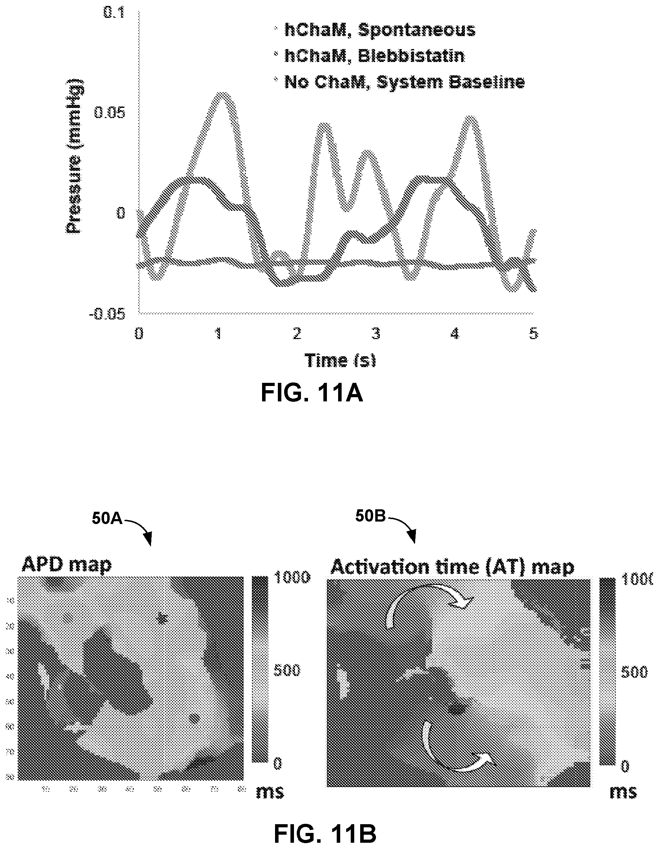

FIG. 11A is a graph of pressure dynamics of an example printed heart using bioink described herein. As shown in FIG. 11A, fluid pressure was measured for a ventricular-like chamber of the printed heart that was created using bioink described herein. The trace with the highest amplitude illustrates spontaneous mechanical activity of the chamber, and the trace with lower peaks is pressure for the same chamber in the presence of blebbistatin to demonstrate inhibited pressure amplitudes in the presence of myosin blockade. The relatively flat line is the system baseline without contraction. Other clinically-important, physiologically complex mechanical parameters can also be measured. FIG. 11B is an optical map of an example printed heart using bioink described herein. As shown in FIG. 11B, electrical parameters including action potential (ADP map of image 50A) and, calcium transients and conduction velocity (Activation time (AT) map of image 50B) are shown for a printed heart using the bioink described herein.

FIGS. 12-44B illustrate another example of bioinks and techniques for creating 3D structures such as a cardiac structure. As discussed herein, a goal of cardiac tissue engineering is the generation of a living, human pump in vitro that could replace animal models and eventually serve as an in vivo therapeutic. Models that replicate the complex structure of the heart, harboring chambers and large vessels with soft biomaterials, can be achieved using 3D bioprinting. Yet, inclusion of contiguous, living muscle to support pump function has not been achieved. One challenge is attaining high densities of cardiomyocytes, a notoriously non-proliferative cell type. One strategy is to print with human induced pluripotent stem cells (hiPSCs), which can proliferate to high densities and fill tissue spaces, and subsequently differentiate them into cardiomyocytes in situ. In this manner, these techniques describe example bioinks capable of promoting hiPSC proliferation and cardiomyocyte differentiation in order to 3D print electromechanically functional, chambered organoids composed of contiguous cardiac muscle. However, these bioinks may also promote the proliferation of other types of cells (i.e., cells other than cardiomyocytes) in other examples.

For example, a photo-crosslinkable formulation of native extracellular matrix (ECM) proteins can make up an example bioink configured to 3D print hiPSC-laden structures with two chambers and a vessel inlet and outlet. After hiPSCs proliferated to a sufficient density, the cells were differentiated within the structure and demonstrated function of the resultant human chambered muscle pump (hChaMP). These created hChaMPs demonstrated macroscale beating and continuous action potential propagation with responsiveness to drugs and pacing, as discussed further below. The connected chambers allowed for perfusion and enabled replication of pressure/volume relationships fundamental to the study of heart function and remodeling with health and disease. These bioinks, and the resulting structures enabled to be generated, may be used to generate macroscale tissues, akin to aggregate-based organoids, but with the critical advantage of harboring geometric structures essential to the pump function of cardiac muscle. In addition, such generated printed structures (e.g., human chambered organoids) of this type might also serve as a testbed for cardiac medical devices and/or therapeutic tissue grafting.

The following techniques were used to test different bioinks and use example bioinks to print and create the structures described with respect to FIGS. 12-44B. As discussed herein, the hChaMPs were composed of an example bioink containing gelatin methacrylate (GelMA), collagen methacrylate (ColMA), fibronectin, laminin-111, and lithium phenyl-2,4,6-trimethylbenzoylphosphinate as a photo-crosslinker. In the following example, to prepare a bioink precursor solution, 2% (weight by volume (w/v)) LAP and 40% (w/v) GelMA were dissolved in mTesR^{™}1 at 60°C for 2 hours. One percent ColMA was dissolved in 20mM acetic acid at room temperature. (Concentrations were varied for ink optimization experiments as described below.) ColMA solution was mixed with GelMA solution at a 1:1 ratio (volume by volume (v/v)) and then incubated at 37°C for 1 hour. Prior to mixing with the cells, 1M sodium hydroxide solution was added to the bioink at a 1:100 ratio (v/v) to neutralize the pH of the bioink. To produce the final cell-laden bioink, the solution was then mixed 1:1 with a suspension of 30 million cells/ml in mTesR^{™}1 containing 187.5 µg/ml LN and FN and 10 µM Y-27632 2HCL ROCK inhibitor. The final solution contained 15 million cells/ml with 10% GelMA, 0.25% ColMA, 93.75 µg/ml LN and FN, 0.5% LAP, and 5 µM ROCK inhibitor.

For measuring viscosity of the bioinks, a series of compositions (as shown in Table 1 below) were prepared without cells. A LVDVII+ cone and plate viscometer connected to a water bath was used to determine the viscosity of the uncrosslinked polymer solutions. The water bath temperature and the printing temperature were both set to 27°C, and the speed of viscometer was 1.5 RPM for all the measurements. The viscosities were recorded once the system reached thermal equilibrium and the reading values did not change more than 0.1 centipoise (cP) over 1 min.

To accommodate the relatively low viscosity of the optimized bioink, 3D printing was conducted using the Freeform Reversible Embedding of Suspended Hydrogels (FRESH) method by which the structure of printed constructs can be maintained with a thermo-reversible gelatin support bath. The gelatin slurry was prepared by dissolving 4.5% (w/v) gelatin type A in 150 ml PBS solution and solidifying it at 4°C overnight, and then the gelatin hydrogel was transferred to a consumer grade blender with 350 ml PBS pre-cooled to 4°C. The contents were then blended in temporal cycles of two seconds on and three seconds off for 3 min at the low-grate setting to disintegrate the hydrogel following by the high-grate setting for 5 min to create homogenized gelatin microparticles. The blended gelatin slurry was then loaded into 50 ml conical tubes and centrifuged at 3000 rpm for 2 min. The supernatant was then discarded, and the precipitated gelatin microparticles were stored at 4°C until use.

hiPSCs overexpressing cyclin D2 (CCND2) under the myosin heavy chain (MHC) gene were maintained in mTesR^{™}1 (Stem Cell Technologies, Vancouver, Canada) and passaged with ReLeSR^{™} every 3-7 days. These cells may be referred to as human cardiac fibroblast-derived induced pluripotent stem cells overexpressing Cyclin D2 (CCND2) under the myosin heavy chain (MHC). To prepare cells for ink optimization experiments or printing of hChaMPs, hiPSCs were dissociated for 8 minutes with Accutase^{®} and resuspended in mTesR^{™}1 with or without FN and LN (always used in combination) with 10 µM ROCK inhibitor to a concentration of 30 million cells/mL. The cell-ECM suspension was subsequently mixed 1: 1 with GelMA/ColMA ink to produce a final cell density of 15 million cells/ml of bioink with 5 µM ROCK inhibitor.

3D printed hChaMPs or pipetted samples for ink optimization were cultured in mTesR^{™}1 supplemented with 5 µM ROCK inhibitor for 24 hours after cross-linking, after which they were cultured in mTesR^{™}1 with daily media changes for 13 more days. After allowing cells to proliferate for 14 days, differentiation was initiated by treating constructs with 12 µM CHIR99021 in RPMI + B-27 Supplement minus insulin. After 24 hours, CHIR media was removed and replaced with fresh RPMI + B-27 minus insulin media. On Day 3, samples were treated with 5 µM IWP-2 in half old/half fresh RPMI + B-27 minus insulin media. On Day 5, the media was changed to RPMI + B-27 Supplement with insulin with subsequent media changes on Day 7 and every three days after that. Starting on Day 20, samples were treated with glucose-free DMEM containing 4 mM sodium L-lactate for 4 days total, with fresh lactate medium added on Day 22. On Day 24, samples were recovered by washing with PBS and replacing media with RPMI + B-27 with insulin, which was then replaced every 3 days until further tests or fixation of tissue constructs.

hiPSCs were dissociated and resuspended at a concentration of 30 million cells/mL in mTesR^{™}1 containing 2x the appropriate concentrations of FN and LN for each ColMA concentration along with 10 µM ROCK inhibitor. After mixing cell suspension 1: 1 with pre-made GelMA/ColMA bioink, the final concentrations of FN and LN were 37.5 µg/ml for 0.1% ColMA conditions, 93.75 µg/ml for 0.25% ColMA conditions, and 187.5 µg/ml for 0.5% ColMA conditions, with 15 million cells/mL of bioink for all conditions. For conditions without FN or LN, cells were resuspended in mTesR^{™}1 alone with ROCK inhibitor prior to mixing with GelMA/ColMA. The resulting cell-laden bioink was deposited into 12 well plates by pipetting 50 µL per well. Samples were cross-linked with a 405 nm flashlight for 20 seconds before the addition of 2 mL mTesR^{™}1 with 5 µM ROCK inhibitor. Maintenance, differentiation, and lactate purification of these constructs were performed as described above. An Axiovert CFL 40 microscope was used to take brightfield images on Day -13 and Day 0 and to take videos of beating after differentiation was complete. Samples were fixed in 10% buffered formalin either post-differentiation or post-lactate treatment so that immunostaining for cardiac troponin could be performed.

Cell viability at Day -13 was assessed for the various bioink formulations described in Table 1 by measuring percent cell area. Single cells were detected in 10x images via edge detection on Fiji. Cell area could then be filled in and measured as a percent of the whole image. Proliferation was assessed by measuring cell colony area on 4x images at Day 0 using thresholding on Fiji to detect colonies and calculating as a percentage of total area.

Three pipetted tissue samples from the optimized formulation were taken from optimization experiments for DNA isolation. Tissue weight was measured, and then a PureLink^{™} Genomic DNA Mini Kit (Invitrogen^{™}, Carlsbad, CA) was used to extract DNA from these samples. Elution volume was set to maximize the DNA yield and the resulting DNA concentration was measured using a Take3^{™} Microvolume Plate and microplate reader (Biotek, Winooski, VT). From this measurement, total DNA weight per tissue weight was calculated.

To understand the viscoelastic properties of the crosslinked, optimized bioink, circular discs of 8 mm diameter and 2 mm thickness were created by adding the optimized bioink into a PDMS mold. Constructs were crosslinked with a 405 nm flashlight for 20 seconds to form hydrogels and then stored in PBS overnight. Rheometric analyses were performed by an AR-G2 rotational rheometer with frequency sweeps performed from 0.1 to 10 rad/s at 1.0% strain at 37°C.

hiPSCs were dissociated and resuspended at a concentration of 30 million cells/mL in mTesR^{™}1 containing 187.5 µg/ml LN and FN and 10 µM ROCK inhibitor. This suspension was then mixed 1: 1 with the bioink precursor solution. The resulting bioink contained 15 million cells/ml with 10% GelMA, 0.25% ColMA, 93.75 µg/ml LN and FN, 0.5% LAP, and 5 µM ROCK inhibitor. hChaMPs were printed on an INKREDIBLE Bioprinter (CellInk, Gothenburg, Sweden) into the aforementioned gelatin microparticle support bath maintained at room temperature. Print pressure was set to achieve optimal ink flow from the needle (27 gauge, 1 inch) at 27°C. While the pressure varied from print to print, it fell within the range of 28 to 38 kPa. hChaMPs were cross-linked on all 6 sides for 20 seconds with a 405 nm flashlight and then incubated at 37°C for at least 30 minutes to allow the gelatin support bath to liquefy. After removal of the gelatin, hChaMPs were washed three times with PBS before being transferred into a 6 well plate with 8 ml of mTesR^{™}1 supplemented with 5 µM ROCK inhibitor. hChaMPs were maintained and differentiated as described above.

An MRI image stack of the heart of a healthy patient was obtained from the Visible Heart Lab at the University of Minnesota. The image stack was segmented, reduced in scale by 10 times, and converted to .stl format using the Mimics software suit. A septal throughway was created between the ventricles in the 3D model to support closed-loop perfusion. An MRI image stack of the 3D printed structure was obtained using a 31cm bore 9.4 Tesla MRI. The MRI image stack was segmented and converted to a 3D model in .stl format using Slicer 4.10.0 software.

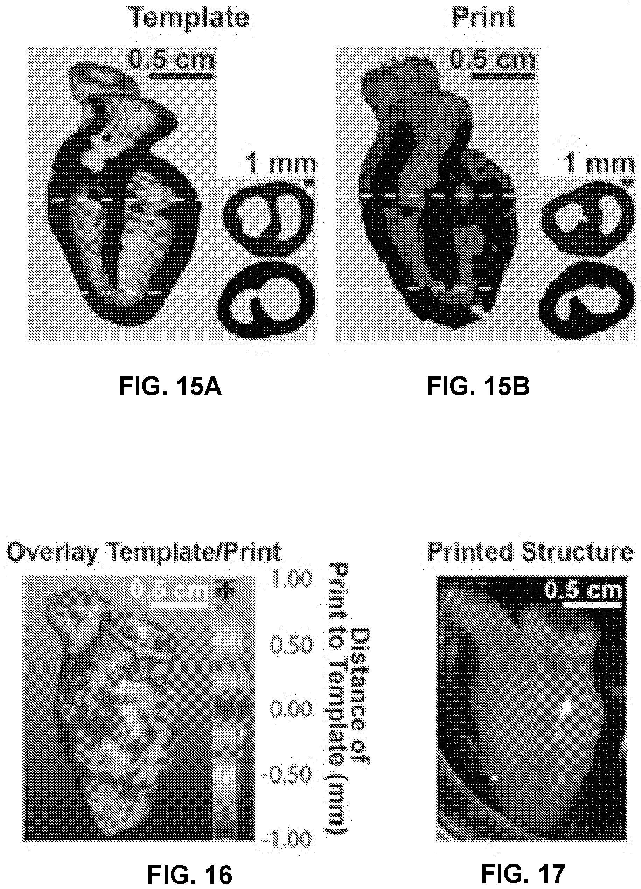

The print fidelity was obtained by comparing the "printed model" with "template model" through CloudCompare^{®} 2.10.2 software (www.cloudcompare.org). Initially, the "printed model" was positioned so it overlapped with the "template model" through manual model shifts. Then, the two models were accurately overlaid using 3D registration with the "template model" as the reference at 40 iterations of 50,000 points. In order to get the most accurate overlap, 3D registration was performed thrice on the models with the "template model" being the reference in each iteration. After registration, the two models were compared using the cloud to mesh tool in CloudCompare, which generated a distance heat map and a histogram of the distance difference between the points of the "printed model" and the "template model" (as shown in FIG. 16). The distance scale in CloudCompare was then calibrated to mm to allow for accurate error measurements. The print fidelity was determined by the percentile of points that fell between a given error margin, which was provided by the histogram generated previously.

Cross-Sectional Area (CSA) was obtained by taking a slice through both the "printed model" and the "template model" using CloudCompare. This slice was filled in using Blender 2.79b software, reimported into CloudCompare, and analyzed using NIH Fiji software.

A simple dye perfusion test was performed to verify that the inner chambers of the hChaMP were connected, leak-proof, and perfusable. The hChaMP was positioned in a 10 cm dish and tubes with 3.175 mm and 1.588 mm outer diameter were inserted into the large and the small vessel, respectively. Extra bioink was deposited at the junctions to secure the tubing. Dye solution was prepared by adding blue food color dye to 50 % glycerol solution at the ratio of 1:500 (v/v). The dye solution was then loaded into a syringe with a 21-gauge needle and injected through the small vessel until the coloring flow effused out from the large vessel.

hChaMPs were fixed in 10% buffered formalin for 24 hours and then dehydrated in 30% sucrose for at least 24 hours prior to embedding in OCT compound. The tissue was sectioned at a thickness of 5-10 µm. For immunostaining of phenotypic and maturation markers, cryosections were fixed with 4% PFA, permeabilized in 0.25% Triton X-100, blocked in Ultra-V Block buffer, incubated with primary antibodies (mouse anti-α-sarcomeric actinin (Sigma-Aldrich, St. Louis, MO), goat anti-CD31 (Santa Cruz Biotech, Dallas, TX), mouse anti-αSMA (Sigma-Aldrich, St. Louis, MO), rabbit anti-cTnI (Abcam, Cambridge, United Kingdom), goat anti-cTnI (Abcam, Cambridge, United Kingdom), rabbit anti-Cx43 (Abcam, Cambridge, United Kingdom), rabbit anti-Ki67 (Abcam, Cambridge, United Kingdom), mouse anti-Kir2.1 (Abcam, Cambridge, United Kingdom), rabbit anti-Bin1 (Abcam, Cambridge, United Kingdom), rabbit anti-RyR2 (Abcam, Cambridge, United Kingdom), and rabbit anti-SERCA2 (Abcam, Cambridge, United Kingdom), washed with PBS, and then stained with corresponding fluorescent secondary antibodies (Jackson ImmunoResearch Lab, West Grove, PA). The nuclei were stained with 4',6-Diamidino-2-Phenylindole (DAPI), and the slides were washed and examined under a FV3000 confocal microscope (Olympus, Tokyo, Japan).

Apoptosis was evaluated with an In-situ Cell Death Detection Kit (Roche Applied Science, Penzberg, Germany) as directed by the manufacturer's instructions. Both the total number of cells and the number of terminal deoxynucleotidyl transferase dUTP nick end labeling-positive (TUNEL⁺) cells were determined, and then apoptosis was quantified as the ratio of the number of TUNEL⁺ nuclei to the total number of nuclei per HPF. Analyses were automated and performed with FIJI software.

For staining of cardiac troponin T (cTnT) in gels and hChaMPs and ECM proteins in hChaMPs, tissues were permeabilized for 1 hour with 0.2% Triton-X and treated with blocking buffer containing 0.1% Triton X-100, 1% glycine, 5% bovine serum albumin, and 2% goat serum for 2 hours. Samples were incubated overnight with primary antibodies (mouse anti-cTnT (ThermoFisher, Waltham, MA or Abcam, Cambridge, United Kingdom), mouse anti-fibronectin (Sigma-Aldrich, St. Louis, MO), rat anti-laminin 1 (alpha and beta chains, Sigma-Aldrich), mouse anti-collagen I (Abcam, Cambridge, United Kingdom), and mouse anti-collagen III (Calbiochem, San Diego, CA)) and then for 2 hours with corresponding secondary antibodies (goat anti-mouse AlexaFluor647 (ThermoFisher, Waltham, MA) and goat anti-rat AlexaFluor647 (ThermoFisher, Waltham, MA)). Samples were co-stained with DAPI.

Calcium movement in hChaMPs was recorded after recovery from lactate treatment using a DMi8 fluorescence microscope (Leica, Wetzlar, Germany). hChaMPs were first cultured with 5 µM Fluo-4 acetoxymethyl ester for 30 min at 37°C and then washed with Tyrode's salt solution for another 30 min at 37°C. After that, the plates with hChaMPs were moved onto the microscope stage and covered with a heating plate to maintain the temperature at 37°C. Fluo-4 AM intensity was recorded at a frame rate of 6.90 Hz with 30 ms exposure time. The acquired data was processed by Fiji and a custom-written script in MATLAB (MathWorks). To generate the calcium traces, background signals of nonactive regions were subtracted from fluorescence signals of beating sites and then normalized against the baseline intensity. The effects of ionotropic drugs on calcium activity of hChaMPs were also analyzed by incubating Fluo-4 AM-treated hChaMPs with 1 µM Isoproterenol or 0.5 µM Verapamil for 10 min at 37°C prior to being imaged. Calcium transients are presented as normalized intensity (f/f₀) over time, along with the average peak amplitude and the average interspike interval.

The hChaMP was immersed in a 10 µM solution of the voltage-sensitive dye di-4-ANEPPS in Krebs-Ringer Buffer for 20 min. After this incubation, half of the dye solution was removed and replaced with RPMI + B27 with insulin media. After 2-5 min stabilization, hChaMPs were excited with a two diode-pumped, continuous-excitation green laser (532 nm, 1 W), and fluorescence intensity was recorded for 15 seconds using 14-bit, 80 × 80-pixel resolution cameras at 500 frames per second. In some hChaMPs, pacing was provided at 1 and 2 Hz via a bipolar electrode and optical mapping movies were recorded during pacing. For drug testing, hChaMPs were treated with 1 µM of isoproterenol and optical mapping movies were recorded with and without pacing.

Optical action potential duration (APD) was measured at 80% repolarization, and two-dimensional (2D) APD maps were constructed to reveal the spatial distribution of APD on the hChaMP surface. Local conduction velocity (CV) was calculated as described previously. Specifically, the distributions of activation times (AT), measured at (dV/dt)ₘₐₓ, for the spatial regions of 3x3 pixels were fitted with the plane, and gradients of activation times gₓ and g_{y} were calculated for each plane along the x- and y-axes, respectively. The magnitude of the local CV was calculated for each pixel as (gₓ² + g_{y}²)^{-1/2}. Mean values for CV were calculated for the visible surface. Macroscale videos were collected with a Moticam 1000 1.3 Megapixel Camera mounted on a Leica S6 D microscope using Motic Images Plus 2.0 software.

Beating videos of hChaMPs were obtained at 4x on an Axiovert CFL 40 microscope at 8.7 frames per second. Beating rates (contraction, relaxation, and beats per minute) were obtained using an open source automated video analysis algorithm⁴ with a macroblock size of 100 × 100 pixels and a maximum movement detection of 50 pixels to accommodate the robust movement of the tissue. For generation of a heat map of contractility over an entire hChaMP, a macroscale video was used with the macroblock size set to 25 × 25 pixels in the video analysis algorithm with a maximum movement detection of 20 pixels due to the lower magnification.

Pulse pressure and stroke volume of hChaMPs were measured using an ADV500 PV system with a PV catheter. The hChaMP was transferred to a 10 cm dish with 20 mL RPMI plus B-27 Supplement with insulin on a heating plate pre-heated to 37°C to ensure the working temperature was always above 30°C. The PV catheter was inserted and positioned close to the apex of hChaMP to obtain clear pressure and volume signals, and the vessel through which the catheter was inserted was tied off to stabilize the construct. Isoproterenol was given by adding 20 µL of 1 mM solution into the dish to achieve the final concentration of 1 µM. Data acquisition was performed at a sampling rate of 5000 Hz, and the parameters used to generate the volume signal were obtained in preliminary pilot measurements including medium resistivity (0.7 ohm·m) and sigma:epsilon ratio (500,000). The volume reading was further calibrated by conducting a dye dilution test. Briefly, acellular hChaMPs were first monitored by the ADV500 PV system while being compressed and released by 1.45 mm, 2.9 mm, and 4.35 mm from the side. After that, acellular hChaMPs were incubated in RPMI + B-27 Supplement with insulin media with 2% Eosin Y overnight and then rinsed with PBS 3 times before being transferred to a 10 cm dish with 20 ml RPMI + B-27 Supplement with insulin. Next, the hChaMP was compressed and released by 1.45 mm depth from the side for 3 times, and the hChaMP was then removed and the bulk medium was collected and examined by a Cytation 3 microplate reader at 524 nm for absorbance from which the displaced volume could be determined. The dye dilution experiments were repeated with the other two compression depths, 2.9 mm and 4.35 mm. The result volume outputs were compared with the values acquired by the ADV500 PV system with corresponding compression depths to determine the calibration factor.

The acquired data was post-processed and analyzed by a custom-written script in MATLAB. In short, a bandpass filter with cut-off frequency 0.1 Hz and 20 Hz was applied to reduce the signal noise. The signal was then converted to the frequency domain by fast Fourier transform, and the beating rate of hChaMPs was determined by the frequency value with the maximum intensity from 3 different segments with at least 50 seconds length. To examine the stroke work generated by the hChaMPs, 5 random contraction cycles were used for creating PV loops and the stroke work was determined by the area in the loops.

This example technique also utilized an inverted geometry with filling as an updated approach for 3D bioprinting of low viscosity bioinks. For example, the hChaMP digital template was derived from an MRI scan of a human heart (Visible Heart Lab, University of Minnesota) that was scaled to the size of a murine heart using the Mimics software suite (Materialise, Leuven, Belgium), such that the longest axis was approximately 1.3 cm. In addition, the septum between ventricles was partially removed such that unidirectional flow could be propagated through the printed structure for ease of nutrient delivery. The chamber outlets in the 3D template were enlarged to facilitate bioprinting and allow connection of catheters during perfusion tests. The resultant digital template was used to guide the remainder of the fabrication process. Since the GelMA-based bioink possesses a liquid form at room temperature, it is not able to fully maintain its shape after deposition and prior to crosslinking. Therefore, a negative mold of the digital template was created to facilitate the 3D bioprinting process via an inverted geometry approach. With this approach, Pluronic^{®} F-127 bioink acts as a supporting scaffold and aids in maintaining the cardiac shape formed by deposition of the bioink prior to crosslinking. The supporting ink consisted of 40% (wt/v) Pluronic^{®} F-127 (Sigma-Aldrich, St. Louis, MO) added to a solution of 90% (v/v) deionized water and 10% (v/v) glycerol and combined via stirring in an ice bath for a minimum of 2 hours. The Pluronic solution was then transferred to a 30cc syringe, sealed with a plastic paraffin film and stored at 4°C until use.

The supporting ink was used to fabricate the inverted model, which was segmented into layers using Slic3r software and converted into G-code before 3D printing. 3D bioprinting was conducted via a custom-built 3D printing system with two independent z-axis heads. Before initiating the bioprinting process, all bioinks, tools, printing stages, syringes, needles, glass substrates, and other related accessories were sterilized. An alcohol lamp was used during the bioprinting process to assist with creating a relatively germ-free environment. The bioink and supporting Pluronic were deposited from two syringes controlled by high precision dispensers through nozzles with inner diameter of 510 µm (21 GA GP.020X.25, EFD). A layer height of 400 µm was used for printing the Pluronic support structures (i.e., inverted geometry) prior to filling with the GelMA-based bioink. After printing was completed, structures were placed in phosphate buffered saline solution (PBS) at 4°C for at least 30 minutes to liquefy and dilute out the Pluronic support material.

MRI and anatomical fidelity analysis may also be completed for the cardiac mimic fabricated with the inverted geometry with filling approach. In order to assess the fidelity of the printed structure to the original template, in this example, an MRI scan was performed on the 3D bioprinted cardiac mimic. Prior to this, the 3D bioprinted cardiac mimic was placed inside tap water in a glass vial, and fixed by placing plastic strips at the bottom of the vial. Imaging was then carried out using an MRI system (16.4 Tesla, Varian/Magnex), while the 3D printed cardiac mimic was placed in a 26 cm bore. The number of scans and views were set to 256 and 128,000, respectively. The MRI image stack was then segmented and converted to a 3D model with a stereolithography (STL) format using Mimics software package. 3D registration of the . stl files between the 3D bioprinted cardiac mimic and the modified digital template was achieved using CloudCompare^{®} 2.10.2 software (www.cloudcompare.org) open source software. CloudCompare was also used to overlay the two 3D models and obtain a distance map along with a histogram of the offset between the template and printed construct for 3 × 10⁵ voxels in 40 iterations. The results indicated that the anatomical difference between the two models, both on the outer surface and inner chambered surface, is not significant, and the fraction of voxels of the printed structure within 0.5 mm of the template was 71.9%, with a peak of distance from print to template close to 0 mm.

A perfusion test may also be performed for the cardiac mimic fabricated with the inverted geometry with filling approach. To assess the perfusability of the 3D bioprinted cardiac mimic, a polyethylene catheter was attached to the inlet of the construct using tissue adhesive. A syringe containing the dye ink was then connected to the other end of the catheter. The dye was injected through the inlet of the 3D bioprinted cardiac mimic to allow for continuous flow from one chamber to the other, and out through the other vessel.

3D bioprinted cardiac mimics printed with the inclusion of hiPSCs were cultured in mTesR^{™}1 (Stem Cell Technologies, Vancouver, Canada) with 5 µM ROCK inhibitor for the first 24 hours after printing and then maintained in mTesR^{™}1. Images were obtained at days 1, 3, 5, and 7 after printing on an Axiovert CFL 40 microscope to assess cell viability.

Described herein are example formulation of extracellular matrix proteins that may support the differentiation of cardiomyocytes from induced pluripotent stem cells. As a part of this experiment, several bioink formulations were evaluated to identify an extracellular matrix-based bioink to support the generation of complex cardiac tissue. The design criteria, in this example, included printability to support extrusion from a 27 gauge needle resulting in a linewidth resolution of 210 µm, easy material handling of the printed structure, the capacity to support proliferation of human induced pluripotent stem cells (hiPSCs) incorporated directly into the bioink, and the ability to support cardiomyocyte differentiation of said hiPSCs.

According to the above example specifics, FIG. 12 is a conceptual diagram of a technique to form example gels with a bioink. As shown in FIG. 12, a gelatin methacrylate (GelMA) base material was obtained to be crosslinked via photoactivation with lithium phenyl-2,4,6-trimethylbenzoylphosphinate (LAP) to accomplish printability requirements and avoid inclusion of synthetic support materials (100). Material handling was difficult below 10% GelMA as final structures were flimsy and easily damaged; whereas printability was difficult to accomplish above 15% GelMA as extrusion required high pressures which made material control difficult and which sometimes resulted in cell damage. Thus, a matrix may beneficially contain either 10% (100 mg/mL) or 15% (150 mg/mL) GelMA, in some examples. To the GelMA, hiPSCs and ECM proteins that may support cardiomyocyte differentiation, namely fibronectin, laminin-111, and collagen methacrylate ranging from 0 - 0.1875 mg/mL, 0 - 0.1875 mg/mL, and 0 - 0.5% (or 0 - 5 mg/mL) respectively, were added to the GelMA (102). Fibronectin and laminin were added in equal amounts for all conditions, with the concentration scaled to the ColMA concentration (0.0375 mg/ml fibronectin and laminin with 0.1% ColMA, 0.09375 mg/ml fibronectin and laminin with 0.25% ColMA, and 0.1875 mg/ml fibronectin and laminin with 0.5% ColMA). In other examples, differing amounts of fibronectin and laminin may be added. All conditions are annotated with GelMA (G) concentration, ColMA (C) concentration, and the presence of the corresponding amounts of fibronectin (F) and laminin (L). For example, 10G0.10CFL refers to a bioink consisting of 10% GelMA and 0.10% ColMA supplemented with fibronectin and laminin. Each formulation material was then added to an array of extracellular matrix (ECM) (104), each formulation was crosslinked via photoactivation (106), and then each formulation as crosslinked was assessed to obtain the values presented in Table 1 below.

**Table 1:**

| Formulation | Viscosity (d-15, cP) | Cell Area (d-13, %) | Colony Area (d-0, %) | Differentiation (d-0, %) | Beating (0-3) |
|---|---|---|---|---|---|
| 10G0.10CFL | 14.3 | 21.8 ± 1.9 | 52.0 ± 3.4 | 46.6 ± 23.9 | 2.8 |
| 10G0.25CFL | 14.7 | 21.6 ± 2.9 | 57.6 ± 7.4 | 67.9 ± 6.6 | 2.8 |
| 10G0.50CFL | 15.9 | 20.3 ± 0.7 | 61.8 ± 3.4 | 17.2 ± 29.8 | 1.6 |
| 15G0.10CFL | 34.2 | 20.9 ± 2.0 | 32.2 ± 21.4 | 11.2 ± 17.4 | 0.8 |
| 15G0.25CFL | 34.9 | 23.3 ± 0.4 | 55.0 ± 7.6 | 23.7 ± 16.2 | 2.0 |
| 15G0.50CFL | 35.6 | 21.3 ± 1.9 | 54.1 ± 9.0 | 35.4 ± 6.4 | 2.8 |
| 10G0.10C | 9.6 | 17.3 ± 1.3 | 55.2 ± 4.3 | 38.2 ± 11.3 | 2.5 |
| 10G0.25C | 11.0 | 16.8 ± 0.2 | 53.5 ± 4.4 | 44.4 ± 7.0 | 2.8 |
| 10G0.50C | 11.9 | 18.9 ± 0.6 | 55.0 ± 3.1 | 42.3 ± 1.6.9 | 2.2 |
| 15G0.10C | 19.6 | 19.2 ± 2.5 | 16.2 ± 11.5 | 0.2 ± 0.5 | 0.1 |
| 15G0.25C | 23.7 | 20.0 ± 1.4 | 38.2 ± 7.3 | 4.4 ± 6.0 | 0.5 |
| 15G0.50C | 24.1 | 17.2 ± 1.7 | 21.0 ± 19.7 | 0 ± 0 | 0 |
| 10G0 | 11.6 | 20.6 ± 0.8 | 51.1 ± 2.7 | 43.5 ± 4.5 | 2.9 |
| 15G0 | 18.2 | 14.7 ± 1.3 | 9.2 ± 3.8 | 0.1 ± 0.1 | 0 |

As shown in Table 1 above, observed example data for different bioink formulations are provided. In the Formulations column, "G" represents gelatin methacrylate, "C" represents collagen methacrylate, "L" represents laminin-111, and "F" represents fibronectin. The preceding numbers for each letter indicates the percentage of weight by volume. For example, "10G0.10CFL" refers to a bioink including 10% gelatin methacrylate (GelMA) and 0.10% collagen methacrylate (ColMA), supplemented with fibronectin and laminin. The label "d-X" indicates how many days had elapsed since deposition before the parameter was measured. For example, viscosity was measured on day 15. The "Beating" scale refers to an assessed grade on the contractile performance of the cardiac tissue generated using the associated bioink formulation (e.g., a zero rating indicates no beating and a value of "3" would indicate be relatively highest beating performance).

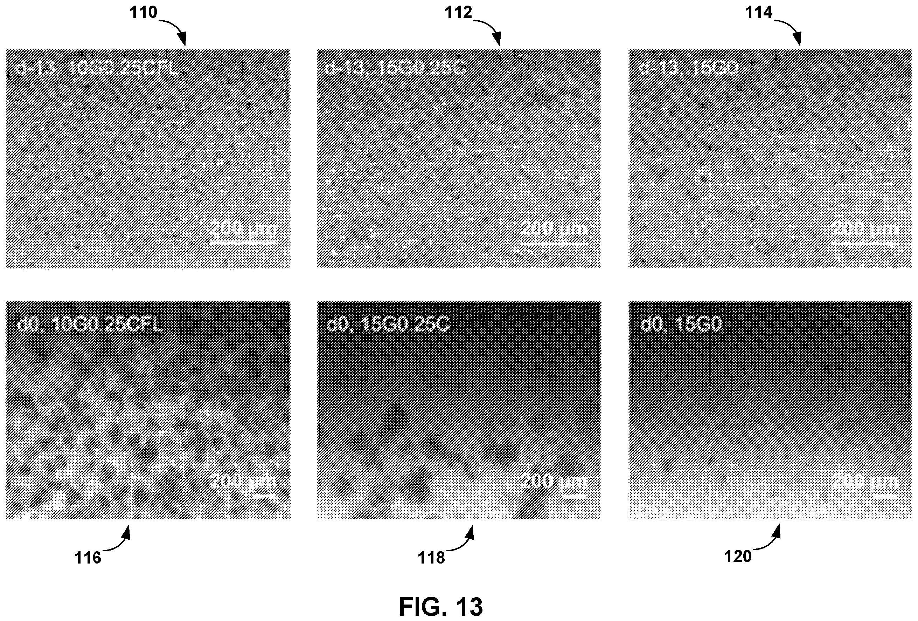

According to design, all of the ink formulations fell within the range of 10-40 cP, ensuring low pressure requirements (28-38 kPa) with printing. In other words, it may be advantageous to select a bioink formulation that has a viscosity from approximately 10 cP to approximately 40 cP. However, some viscosities below or above this range may be appropriate for some applications and structures. Ability to support hiPSC health was assessed via measurement of cell area in the printed structure at Day -13 as an indicator of cell viability and colony area at Day 0 as an indicator of proliferation with continued cell health. We found that viability was relatively consistent across all formulations at Day -13 with the exception of the ink composed exclusively of 15% GelMA. As shown in FIG. 13, cell area coverage for respective different bioink formulations varied. Sample 110 shows relatively good coverage for 10G0.25CFL, sample 112 shows relatively fair coverage for 15G0.25C, and sample 114 shows relatively poor coverage for 15G0.

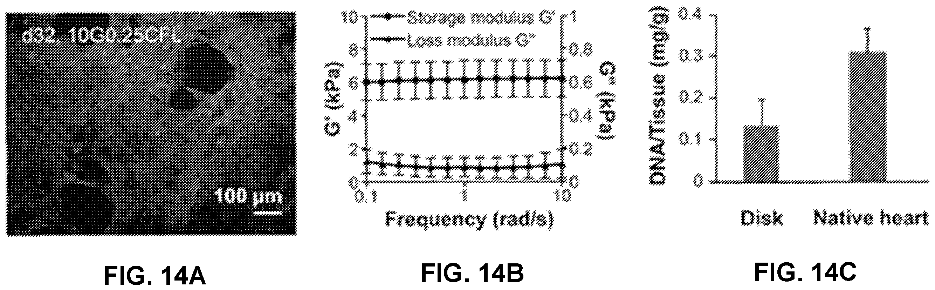

However, proliferation varied substantially across formulations with those gels containing 10% GelMA generally exceeding those with 15% GelMA. As shown in FIG. 13, sample 116 shows relatively good proliferation for 10G0.25CFL, but sample 118 of 15G0.25C and sample 120 of 15G0 both show relatively poor proliferation. It is noted that even the poor samples may be sufficient to support a printed structure in some conditions. Specific formulations 10G0.25CFL, 10G0.50CFL, and 10G0.10C supported the highest levels of proliferation (range, 55.2 - 61.8% colony area). The ability to support hiPSC differentiation to cardiomyocytes was assessed via measurement of cardiac troponin T (cTnT). Differentiation outcomes not only corresponded to the GelMA fraction, but also were dependent on the addition of fibronectin and laminin. For example, as shown in FIG. 14A, the 10G0.25CFL formulation indicated relatively good cardiomyocyte differentiation at day 32 post differentiation. Specific formulations 10G0.10CFL, 10G0.25CFL, and 10G0.25C generally supported the highest levels of differentiation. Although several different formulations may be suitable for a bioink, one formulation ranked high in the top three for all criteria, 10G0.25CFL or 100 mg/mL gelatin methacrylate (GelMA), 2.5 mg/mL collagen methacrylate (ColMA), 95 µg/mL fibronectin (FN), 95 µg/mL laminin-III (LN), and 5 mg/mL lithium phenyl-2,4,6-trimethylbenzoylphosphinate (LAP). For the crosslinked gels of this specific formulation, the storage modulus (G', 6.14 ± 1.13 kPa) was nearly two orders of magnitude higher than the loss modulus (G", 0.09 ± 0.06 kPa) (FIG. 14B), suggesting the elastic nature of the gels with a stiffness similar to that of the late embryonic heart. In addition, this formulation achieved a cell density of 0.1 mg DNA/g of gel (native cardiac tissue, approximately 0.3 mg DNA/g of myocardial mass)³¹ (as shown in FIG. 14C). Therefore, this cell density may be similar to that of cardiac tissue. Therefore, this formation of 10G0.25CFL was subsequently used for all subsequent experimentation and analysis.

However, it is noted that several different formulations of bioinks may be configured to promote printing of structures. For example, example bioink formulations may have a percentage of GelMA from approximately 10 percent to approximately 15 percent. The example bioink may also have a percentage of ColMA from approximately 0.1 percent to approximately 0.5 percent. Although fibronectin and laminin may be included in the bioink, the bioink may still enable printing of cells without fibronectin and/or laminin in other examples.

As shown in FIG. 16, a bioprinted chambered cardiac mimic with large vessel extensions exhibits high fidelity to the digital template. The selected bioink (10G0.25CFL) could be extruded from a needle, but the bioink was of low relative viscosity (14.7 cP) and so the ability to fabricate structures beyond simple stacking geometries can be challenging, although maybe possible, with standard extrusion modalities in air. Therefore, printing was achieved with a technique that involved backfilling an inverted geometry made of sacrificial ink. Complex structures could be generated with this modality; however, low cell viability and low proliferation associated with the sacrificial ink limited further use. Ultimately, printing involved freeform reversible embedding of suspended hydrogels with success, wherein the embedding material or "slurry" consisted of gelatin microparticles. This bioprinting approach was thus used for the remainder of the example described herein.

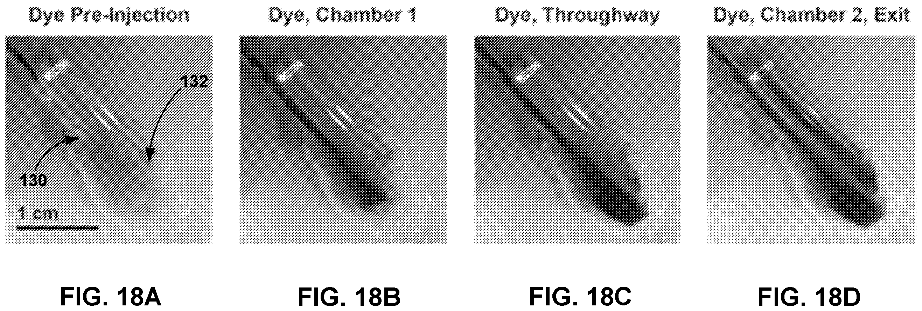

The print template (FIG. 15A) was derived from an MRI scan of a human heart that was scaled to the size of a murine heart such that the longest axis was approximately 1.3 cm. In addition, the septum between ventricles was partially removed to provide a throughway such that unidirectional flow could be propagated through the printed structure for ease of nutrient delivery. The size of the gelatin particles was most critical among print parameters for attaining feature sizes necessary to replicate the chambered cardiac mimic. The size of gelatin microparticles scaled inversely with the resolution of the print. Particles of approximately 100 µm could support printing of the chambered cardiac mimic with intact chambers, patent large vessels, and material properties conducive to handling, as shown in FIG. 15B. MRI scans of the bioprinted structures show intact chambers and volumetric, 3D digital reconstructions of the bioprinted cardiac mimic were compared with the volumetric 3D digital template using CloudCompare^{®} 2.10.2 software (www.cloudcompare.org). A qualitative, cross-sectional comparison showed a high level of fidelity of the interior chambers (FIG. 15A and 15B), which was substantiated quantitatively via generation of a distance map from the overlaid template and printed structure (FIGS. 16 and 17). The heatmap of FIG. 16 shows the geometric difference between the template and the print at a scale of +1.0 mm to -1.0 mm from the template. The fraction of voxels of the printed structure within 0.5 mm of the template was found to be 86%. Further, polyethylene tubing and fluorinated ethylene propylene could be securely attached to the printed structure using tissue adhesive, and inclusion of dye in the perfusate showed intact and perfusable chambers in the interior space of the bioprinted, chambered cardiac mimic as shown in FIGS. 18A-18D. For example, in FIG. 18A, fluid entered the inlet 130, passed through the chamber, and exited the outlet 132 of the hChaMP. FIG. 18D illustrates the dye exiting the chamber from outlet 132.

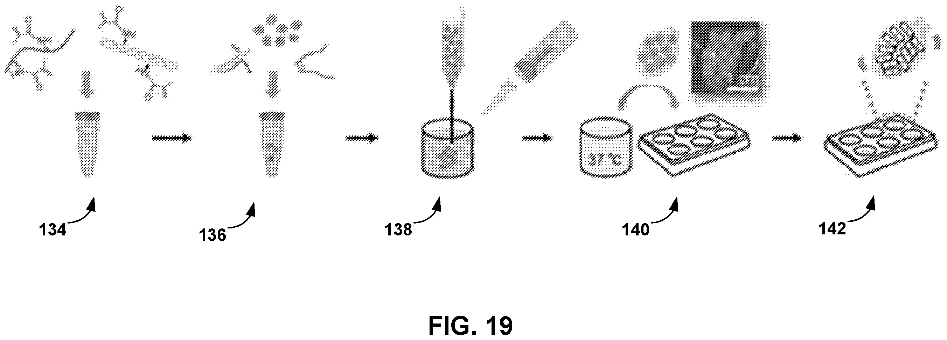

hiPSCs 3D printed in an optimized bioink give rise to a contiguous muscle wall to form a human chambered muscle pump (hChaMP). To determine whether human pluripotent stem cells could proliferate to populate the entire hChaMP, hiPSCs were included during the print using the sample bioink formulation as shown in FIG. 19. For example, the GelMA and ColMA formulation was mixed (134), hiPSCs, laminin, and fibronectin were added (136), and the resulting formulation was bioprinted into the form of the hChaMP and photoactivated (138). Next, the hChaMP is removed from a warm water bath and added to a dish (140) where differentiation was induced and the resulting structure was assessed for performance as described herein (142).

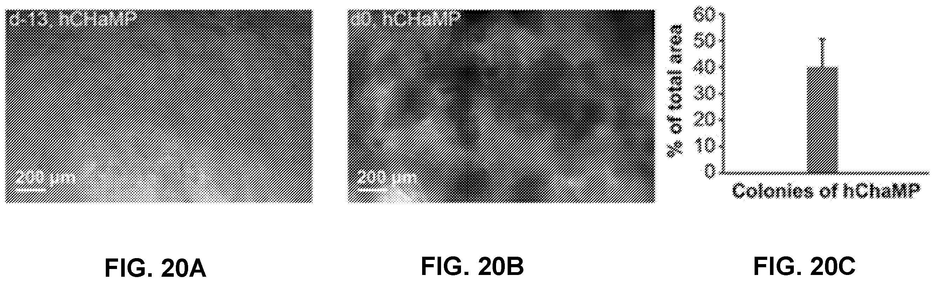

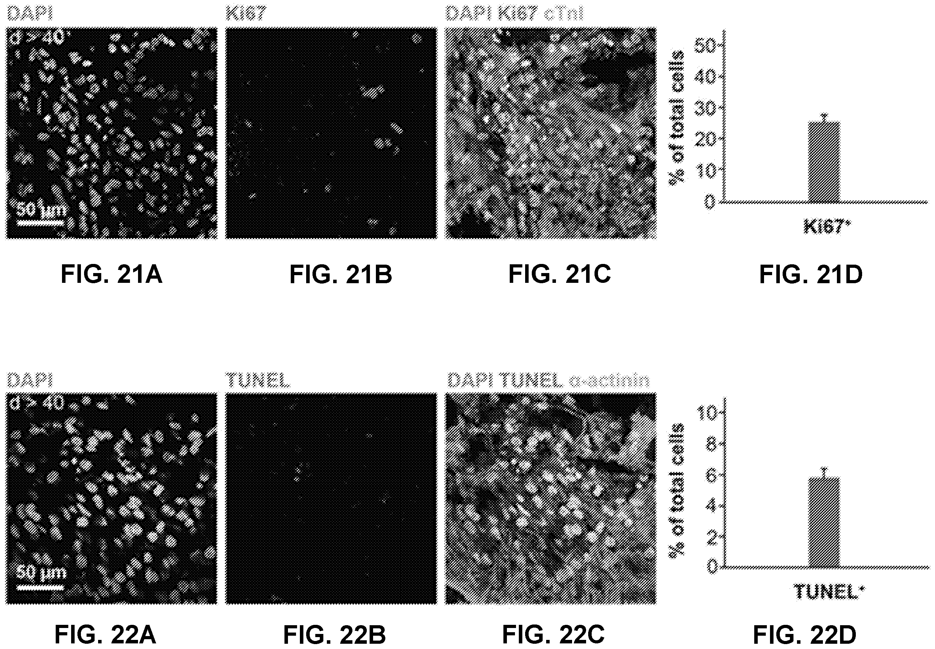

Given the imaging complexity of counting individual cells in such a large construct, we instead quantified colony size in more than four randomly chosen regions of eight different hChaMPs at two weeks in culture. By Day 14, approximately 90% of the bioink volume was populated with cells, both singular and in large colonies, with 40% ± 11% corresponding to colony area, as shown in FIG. 20A which indicates single hiPSCs at day -13 compared to cell proliferation with hiPSC colonies at FIG. 20B. FIG. 20C indicates the percentage of area covered by the colonies at this stage. It was at this point that a cardiomyocyte differentiation protocol was imposed, as shown in FIG. 1B. Six weeks following lactate purification, cells of hChaMPs were again probed for proliferation, this time via expression of Ki67 shown by FIGS. 21A, 21B, and 21C. Approximately 20% of cells are proliferative at this stage, as shown in the immunofluorescence staining via Ki67 in FIG. 21D. At the same time point, cell death was assessed via terminal deoxynucleotidyl transferase dUTP nick end labeling (TUNEL) and showed very limited cell death (e.g., via staining in FIGS. 22A, 22B, and 22C and the percentage of dead cells indicated in FIG. 22D), suggesting ongoing cell health in the hChaMP.

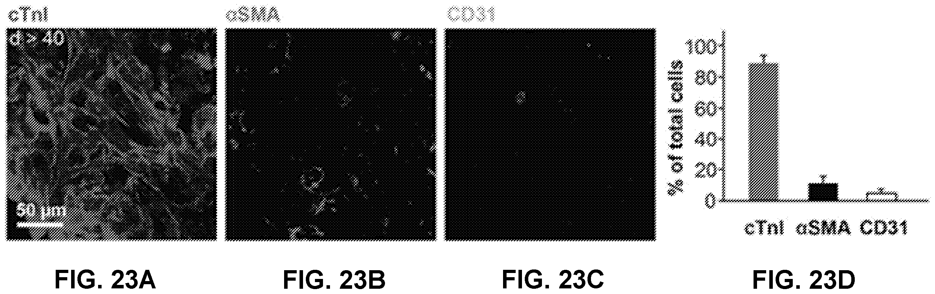

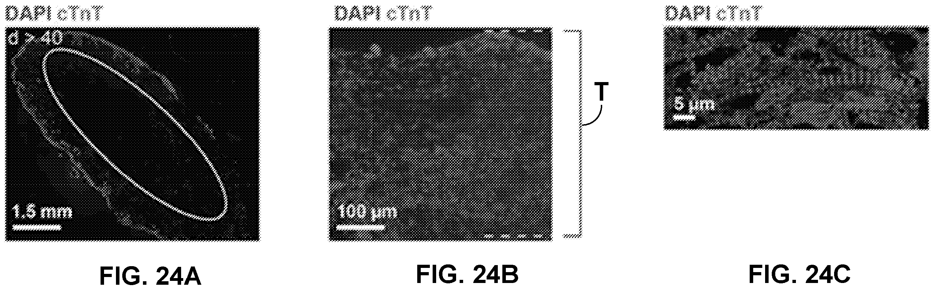

To determine whether hiPSCs could undergo efficient differentiation to cardiomyocytes in the hChaMP and thereby form a contiguous muscle volume, hChaMPs were stained for sarcomeric protein, cardiac troponin I (cTnI). As shown in FIG. 23D, nearly 85% of the cells of the structure were cardiomyocytes according to expression of cTnI (FIG. 23A). Other cardiac cell types may also be used in this process, since mesoderm induction could have been followed by differentiation to cardiac fibroblasts, endothelium, or smooth muscle cells. There was no compelling evidence of cardiac fibroblasts (via staining for discoidin domain receptor 2, DDR2, data not shown), but smooth muscle cells (via staining for alpha smooth muscle actin, αSMA as shown in FIG. 23B) and endothelial cells (via staining for CD31, as shown in FIG. 23C) were present in hChaMPs. The combined cardiac cell cocktail often fully circumvented the hChaMP, as shown in FIG. 24A, and the thickness of the wall was typically between 100 µm and 500 µm with the thickest region exceeding 500 µm, thickness T shown in FIG. 24B. FIG. 24C shows a magnified portion of the wall of FIG. 24B, the magnified portion indicating sarcomeric striations within the hChaMP. The majority of the cells at six weeks following lactate purification were associated with the outer portion of the hChaMP wall. Bioink could be discerned in the inner portion of the hChaMP wall, but was largely without cells as shown in the white oval of FIG. 24A.

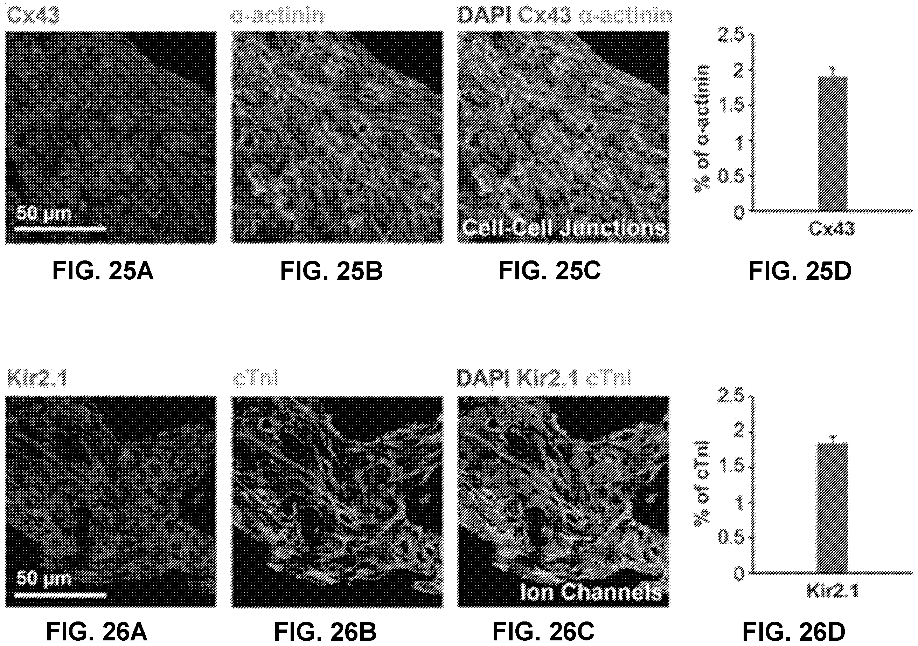

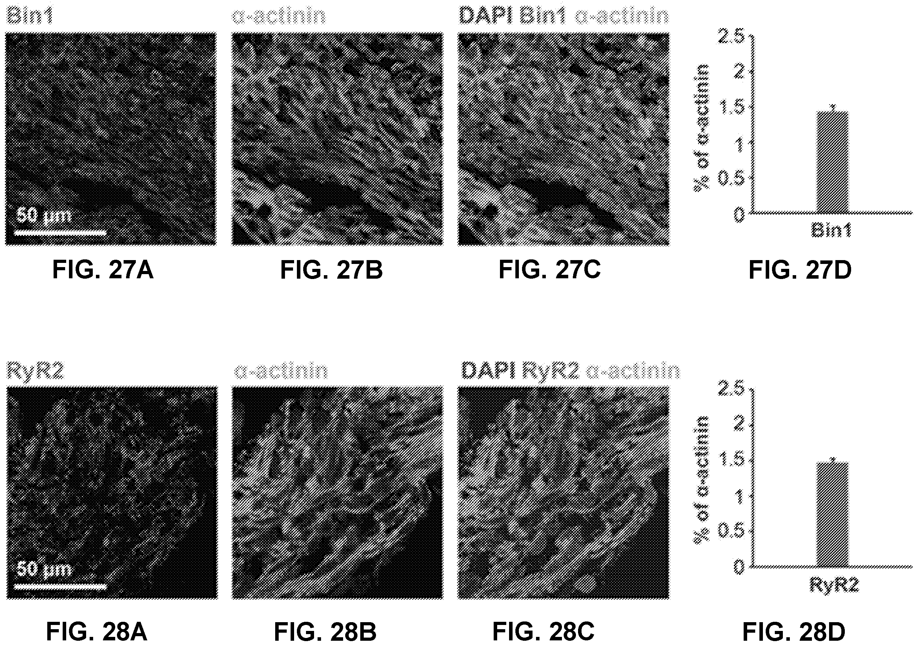

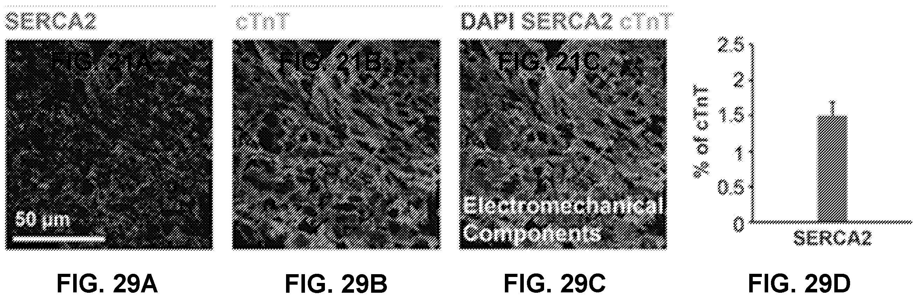

The printed hChaMPs express and localize proteins consistent with a maturing cardiomyocyte phenotype. hChaMPs were cultured for several weeks following completion of the differentiation and lactate purification protocols. Prior to extended culture, hChaMPs were transitioned from static cultures to convective environments wherein nutrient exchange might be better achieved via rocking. Initiating dynamic culture prior to initiating differentiation did not improve the quality of the hChaMP in terms of thickness, purity, or function of the cells of the wall. hChaMPs were neither perfused in a bioreactor, exposed to controlled, resistive mechanical stimulation, nor exposed to electrical stimulation; all factors shown to improve maturation. Even so, robust expression of gap junction protein Connexin 43 (Cx43) was found in substantive plaques between adjacent cardiomyocytes. Also at the cell surface, inward rectifying potassium channel Kir2.1, expressed in fetal and adult cardiomyocytes to stabilize the resting membrane potential, was detected at high levels as shown in FIGS. 25A-25D and 26A-26D. FIGS. 25D and 26D indicate the quantity of Cx43 and Kir2.1 as a percentage of α-actinin and cTnl, respectively. Intracellular machinery associated with the sarcolemma and sarcoplasmic reticulum was also well expressed and appropriately organized, as shown in FIGS. 27A-27D. In particular, bridging integrator-1 (Bin1), a membrane scaffolding protein essential for the formation of T-tubules and their associated function, was found in positive striations along myofibrils. FIG. 27D indicates that Bin1 was about 1.5% of α-actinin. Sarcoplasmic reticulum ATPase2a (SERCA2a) exhibited signal in the majority of cells as shown in FIGS. 29A-29D, with staining in close apposition to the myofibrils. Similarly, ryanodine receptor 2 (RyR2) formed a ladder-like and regular network along the myofibril with greater regularity than that observed in cardiomyocytes derived in a 2D monolayer at similar time points following differentiation as shown in FIGS. 28A-28D, suggestive of advanced maturation in the hChaMP.

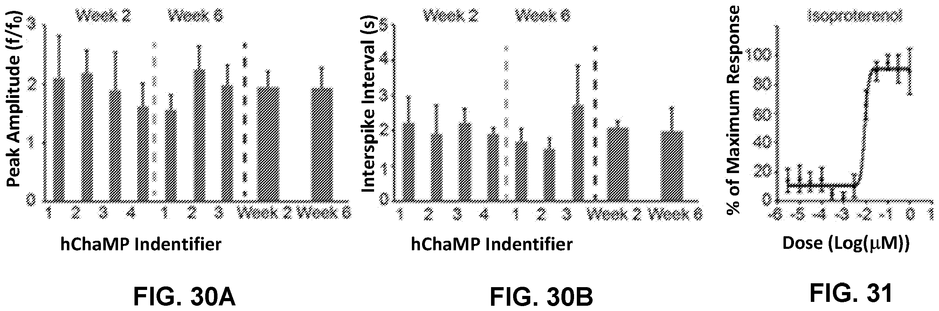

In addition, the hChaMPs exhibited contiguous electrical function and pump dynamics. hChaMPs could be routinely fabricated such that macroscale beating was observed. To determine the extent to which electromechanical function was preserved throughout the complex structure, electrical function was first measured via calcium transients of randomly selected regions of hChaMPs (n ≥ 3 hChaMPs, n ≥ 3 regions per hChaMP, shown in FIGS. 30A and 30B). Calcium transients were measured at both two and six weeks after cessation of the differentiation and purification protocols to determine dynamics of calcium handling over time. Individual hChaMPs showed some degree of variability in both peak amplitude (FIG. 30A) and interspike interval (FIG. 30B) (as denoted by the error bars), likely corresponding to the heterogeneity of the construct with respect to density of cardiomyocytes per unit volume and relative to other cardiac cell types (or non cardiac cell types, though these were of low number). In addition, peak amplitude did not change over time, nor did interspike interval, suggesting that the degree of maturation had largely been achieved by two weeks and that health of the hChaMP could be maintained long term. Calcium handling could be increased in frequency with the addition of isoproterenol, a non-selective β adrenoreceptor agonist and could be decreased in amplitude with the addition of verapamil, a phenylalkylamine calcium channel blocking agent supporting the capacity of the hChaMP to appropriately respond to drug stimuli. In addition, as shown in FIG. 31, a dose response curve was generated from calcium transient data to examine the effect of isoproterenol concentration on beat rate. This dose-response effect of isoproterenol in hChaMPs was measured by calcium transient activity in terms of beat rate, from 5 different areas across two different hChaMPs. From this curve, the isoproterenol concentration that produced 50% of the maximum response (EC50) was determined to be 0.009 µM, which is very similar to what has been previously reported for stem cell derived cardiomyocytes.

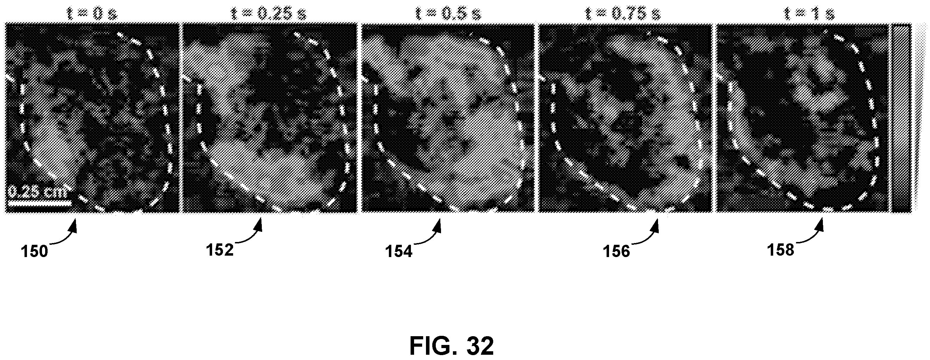

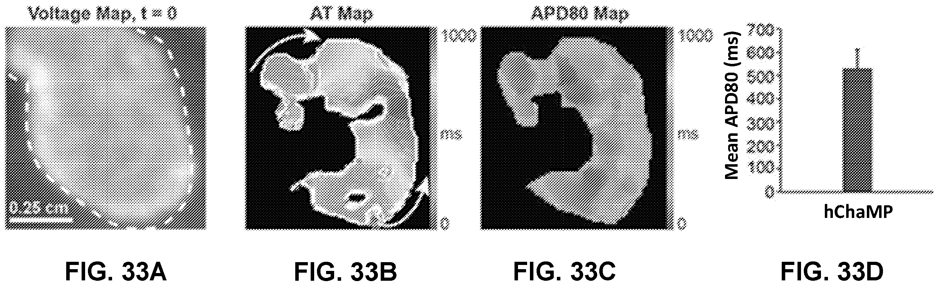

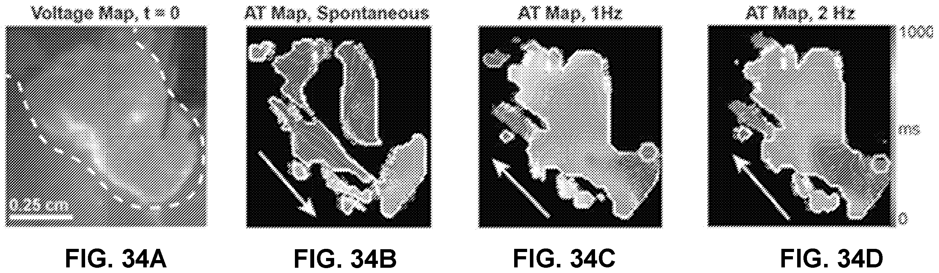

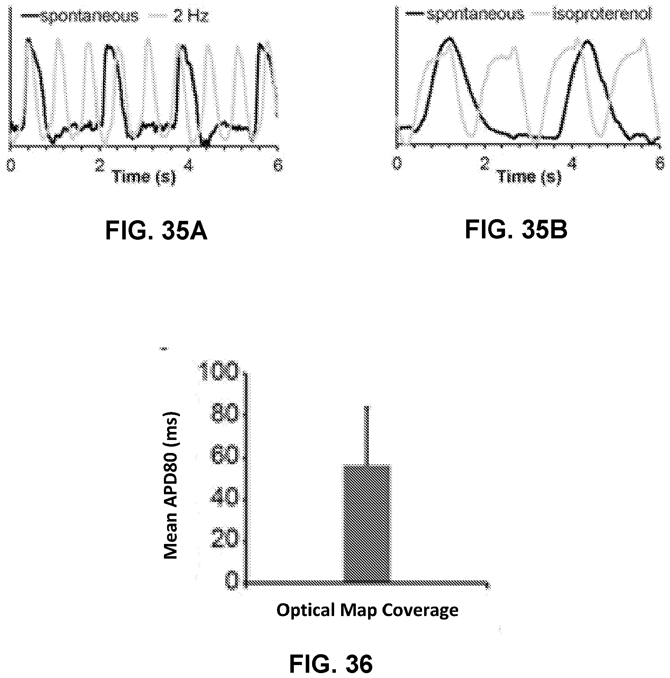

Optical mapping was next used to measure voltage changes throughout the entire hChaMP structure (n ≥ 3 hChaMPs, FIGS. 32-35B). As shown in FIG. 32, this optical mapping provided visualization of electrical signal propagation throughout the hChaMP in real-time, as shown in the progression through images 150, 152, 154, 156, and 158. In most cases, the electrical activity of the hChaMP began in one area and propagated throughout the structure. The location of the structure from which the activity was propagated was stochastic, sometimes from the large vessels, sometimes from a region near the large vessels and sometimes near the apex. This outcome likely reflects the accumulation of pacemaker cells or immature cardiomyocytes with the capacity for spontaneous membrane depolarization in a given region that dominates and therefore initiates the response. FIG. 33A indicates spontaneous electrical activity of hChaMP including activation time at 50% of repolarization (AT) (FIG. 33B) and action potential duration at 80% of repolarization (APD80) in FIG. 33C. FIG. 33D indicates the average time in milliseconds for the APD80 with spontaneous activity from three different hChaMPs. However, in some cases the spontaneous source of depolarization could be overcome and the directionality of propagation altered via electrical point stimulation at another location within the hChaMP as shown in FIG. 34A, and FIGS. 34B, 34C, and 34D illustrate the propagation of stimulation in the direction of the respective arrows. In addition, the hChaMP responded in a dramatic and predicted fashion to altered pacing frequency (FIG. 35A) and drug stimulation with isoproterenol (FIG. 35B). Of note, pacing for less than an hour increased the total area in which action potentials were detected, suggesting electrical stimulation can promote connectivity and further enhance the concerted function of the hChaMP. In the absence of electrical stimulation, FIG. 36 indicates that action potentials were detected across 56% ± 28% of the total surface area of the hChaMP. The locations of detectable electrical signals likely correspond to the distribution of cardiomyocytes throughout the hChaMP, as co-staining of calcium dye-treated tissues confirms that electrically active regions are cTnT-positive.

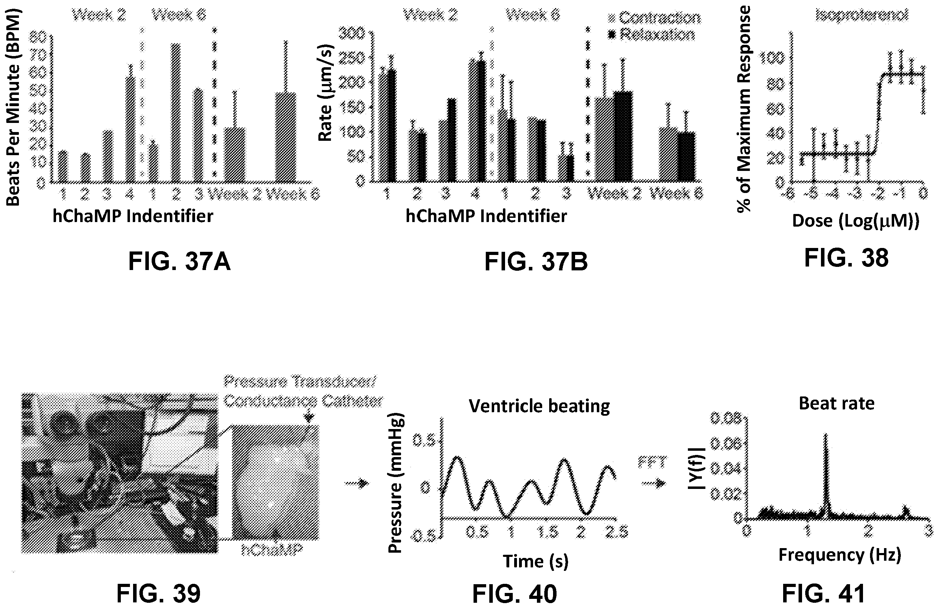

To determine mechanical pump function, hChaMPs were first evaluated for contractile performance. Beats per minute and rates of contraction and relaxation did not vary much within individual hChaMPs but did vary quite substantially between hChaMPs, likely reflecting the relative number of cardiomyocytes and wall thickness per hChaMP. FIGS. 37A and 37B illustrate the variation of beats per minute and rate of contraction and relaxation, respectively, between different instances of the same hChaMPs. The right two bars of each of FIGS. 37A and 37B indicate variability between different hChaMPs. In addition, contractile performance was not enhanced over time but did not decline significantly either, supporting the long-term health of the hChaMP. Contractility analysis was also used to measure the dose response of hChaMPs to isoproterenol treatment, as shown in FIG. 38. The dose-response effect of isoproterenol in hChaMPs was measured by contractility in terms of beat rate, from at least 3 different areas across 2 hChaMPs. This analysis produced an EC50 of 0.009 µM, identical to the value derived from calcium transient analysis.

To determine pressure volume dynamics as a clinically relevant comparator for this new model system shown in FIG. 39, a conductance catheter harboring a pressure transducer was inserted into one chamber of the hChaMP. FIG. 39 shows an overview of the example methodology for obtaining interchamber pressure and volume using a pressure transducer coupled to a conductance catheter for obtaining estimates of distance from the catheter to the chamber wall and back-calculating chamber volume. Catheters were threaded through one of the large vessels extending from the top of the hChaMP and placed submerged in a 37°C bath containing culture medium that was mounted on a temperature-controlled heated stage. Catheter readouts were amplified to provide real time measurement of pressure and volume. Fast Fourier transform was used to convert pressure vs. time associations (FIG. 40) to beat rates. The intra-chamber pressure and volume dynamics were performed over a 2.5 second interval with and without isoproterenol for generating corresponding pressure-volume loops. Beat rate was determined via fast Fourier transform of pressure vs. time plots, as shown in FIG. 41.

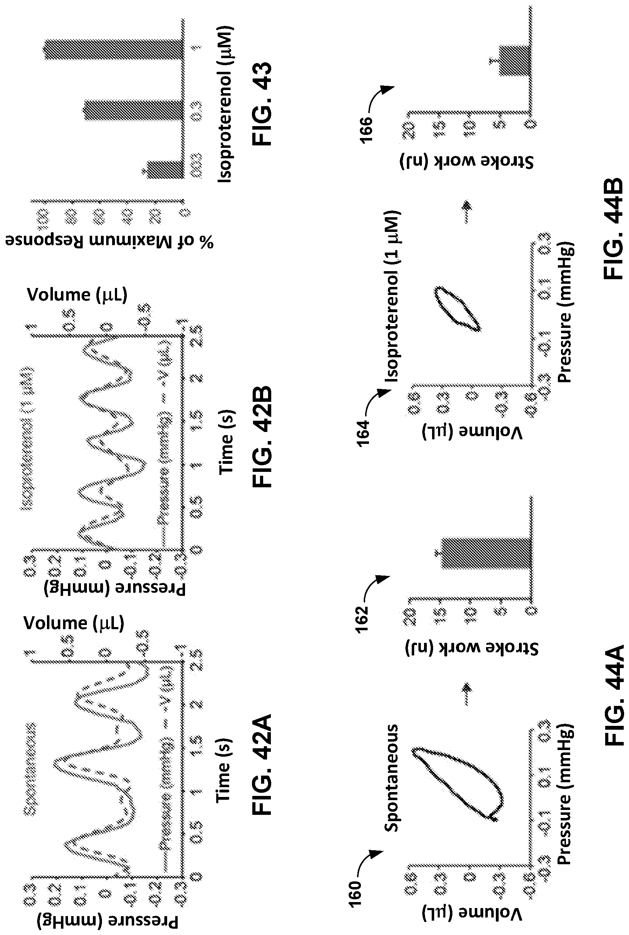

The coupling of the pressure transducer with the conductance catheter enabled plotting of both pressure and volume simultaneously as a function of time, which was done for spontaneously contracting and isoproterenol-treated hChaMPs, as shown in FIGS. 42A and 42B. As shown in FIG. 43, changes in beat rate corresponding to multiple concentrations of isoproterenol were detected using this setup. Isoproterenol response in terms of beat rate was measured by catheters, from 3 fragments spread throughout the sample trace. Pressure-volume vs. time plots were used to generate pressure volume, and from these stroke work (e.g., FIGS. 44A and d44B) could be determined despite the fact that there are no valves to resist emptying and filling in the respective hChaMPs. Graph 160 of FIG. 44A indicates the pressure-volume loop for spontaneous contraction of the cardiomyocytes, and graph 162 indicates the stroke work of each contraction. Similarly, graph 164 of FIG. 44B indicates the pressure-volume loop for isoproterenol induced contraction of the cardiomyocytes, and graph 166 indicates the stroke work of each contraction. When compared to graph 162, stroke work in this case was reduced in the presence of isoproterenol as shown in graph 166. Stroke work was obtained from 5 different contraction cycles. The usual volume moved through the chambers was 0.5 µL and maximum volume moved through the chambers was 5.0 µL, which is approximately 25% of that of the average stroke volume of an adult murine heart. These data indicate that the printed cardiac structures created using the bioink formulations and stem cells can function as fluid pumps.

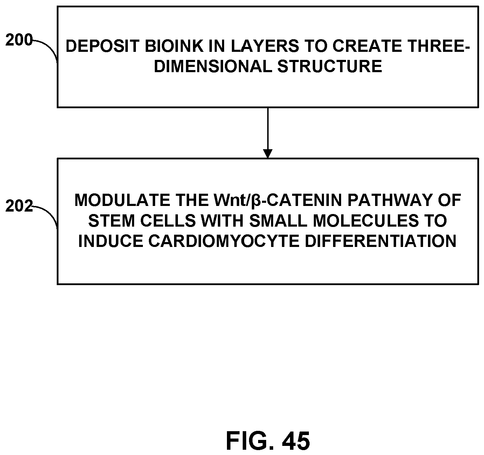

FIG. 45 is a flow diagram of an example 3D printing method using a bioink composition described herein. Any of the example bioinks described herein may be used in this method, and alternatives to this method may also be used. For example, a bioink containing approximately 10 percent weight by volume of gelatin methacrylate, approximately 0.25 percent weight by volume of collagen methacrylate, and approximately 0.5 percent weight by volume of the lithium phenyl-2,4,6-trimethylbenzoylphosphinate can be mixed thoroughly with a solvent such as a solvent containing approximately 74 percent weight by volume of mTeSR medium, 20 percent weight by volume of 20mM acetic acid, and 1 percent weight by volume of 1M NaOH. This solution can then be added to a suspension of human induced pluripotent stem cells (e.g. on a 1:1 ratio) containing approximately 0.19 mg/mL fibronectin and 0.19 mg/mL laminin-111. The final concentrations of fibronectin and laminin-111 may be approximately 93.75 µg/mL each.

As shown in FIG. 12, the prepared bioink composition is deposited (e.g., printed) in layers to create a desired three-dimensional structure (200). This method of depositing includes printing a three-dimensional structure using the bioink to create at least one chamber (e.g., one or more chambers of a heart) and/or other vessels. In some examples, the bioink may be deposited via a syringe into a support bath. In other example, one or more coaxial nozzles may dispense the bioink such that the bioink exits one orifice of the coaxial nozzle and a cross-linking agent simultaneously is dispensed from another orifice of the coaxial nozzle. Dispensing from the coaxial nozzle may be performed prior to, or during, application of light to the dispensed material (if the cross-linking agent is photoactivatable). After printing, the process may include modulating the Wnt/-catenin pathway of the stem cells with small molecules to induce cardiomyocyte differentiation (202). Although the bioink is described in terms of printing herein, disposition of the bioink may be performed via any techniques that may put the bioink in an appropriate position within a matrix, such as via deposition, pipetting, injection, or even molding, casting, or forming the bioink. In this manner, the bioink may refer to a substance that can be deposited into a form to create a desired structure of cells.

Although the support bath may be removed after at least some differentiation, the support bath may be removed prior to differentiation in other examples. In other example, a sacrificial solid material may be used in place of the support bath (or slurry). In this case, the bioink, which may have a relatively low viscosity, may be applied to the sacrificial solid material, and then the solid material may be removed from the printed structure via mechanical, thermal, and/or chemical processes.

As described herein, differentiation of functional cardiomyocytes to populate centimeter-scale, complex structures can be attained via printing of a bioink, representing a critical step toward macrotissues capable of replicating pressure/volume relationships critical to the study of heart function with health and disease. One example bioink may include 10% weight by volume of gelatin methacrylate, 0.5% weight by volume of lithium phenyl-2,4,6-trimethylbenzoylphosphinate, 0.25% weight by volume of collagen methacrylate, and a solvent comprised of 74% mTeSR medium, 20% 20mM acetic acid, and 1% 1M NaOH that has high printability and also allows for growth and proliferation of stem cells. In some examples, the mTeSR medium may include DMEM/F12 basal media supplemented with components such as insulin, selenium, transferrin, ascorbic acid, FGF2 (bFGF), and/or TGFP or nodal, having the pH adjusted with NaHCO3.

Cardiac tissue engineering can use robust differentiation protocols for human cardiomyocytes and microfabrication techniques to generate microscale model systems for drug testing. Expansion to macroscale models, where human heart structure and function can be examined on multiple scales, may support medical device testing, preclinical cardiology, and push research closer to clinical transplantation. Here a macroscale chambered model of the human heart structure may be created by combining basic scientific discoveries in ECM-stem cell dynamics, technical advances in 3D bioprinting and lessons learned from human organoid culture. The ECM-exclusive nature of the bioink means remodeling can occur unencumbered by foreign materials. Indeed, the epitopes provided in the bioink engage several integrin heterodimers including α1β1, α2β1, α10β1, α11β1 (collagen), α5β1, αvβ3 (gelatin), α6β1, α7β1, α6β4 (laminin-111), α4β1, α5β1, ανδ αvβ3 (fibronectin). Of these, hiPSCs express receptors for all ECM of the bioink, namely α11β1, α5β1 and α6β1. Engagement of hiPSCs to the ECM was favored insofar as it promoted cell viability, pluripotency, and anchorage of growing colonies. Striking a balance between maintenance of pluripotency and initiation of differentiation is challenging in the presence of ECM, as only α6β1 has been reported to promote pluripotency, while all others appear to promote differentiation in an ECM type-specific manner. In an attempt to improve hiPSC proliferation, the hiPSCs were preincubated with laminin-111 (binds α6β1) prior to inclusion with remaining components of the bioink. In addition, high cell densities were employed to allow cell-cell interactions to dominate (though not eliminate) cell-matrix interactions. Also, as colonies grew, either ECM hydrolysis or direct remodeling of ECM via matrix metalloproteinases (MMPs) occurred to provide space, as hiPSCs expressed MMPs 1, 5, and 6, where MMP1 is a protease capable of degrading both collagen and gelatin. This expansion phase enabled final cell densities (approximately 0.1 mg DNA/g of hChaMP) of the same order of magnitude as native tissue (approximately 0.3 mg DNA/g of myocardial mass). Over time, other ECM proteins emerged in the hChaMP, most notably type III collagen, which will engage at least integrins α1β1 and α2β1 which are highly expressed in iPSC-derived cardiomyocytes. In addition, iPSC-derived cardiomyocytes express a myriad of MMPs including 1-3, 10, 11, 14-16 and 19, as do hiPSC-derived smooth muscle cells, which were present, albeit more rarely in the hChaMP. Productive remodeling of a living pump of this type may improve functional performance and therefore potential as a model system and future therapeutic relevance.

In some examples, it may be beneficial to increase the thickness, homogeneity and organization of the muscle wall as well as to spur maturation of individual cardiac muscle cells. Increased muscle thickness may improve pump function and prevent rupture. One approach for enabling thick tissue cultivation may be to introduce convective flow in a bioreactor system. For instance, culture of engineered cardiac bundles with neonatal rat ventricular myocytes under dynamics conditions for two weeks can result in a 2.5-fold larger muscle area compared to static conditions. In addition, an average 1.8-fold increase in thickness can occur from self-assembled tissues with adipose-derived stem cells cultured on a rocking platform compared to static conditions. Of note, this same platform showed minimal effects on the tissues formed by fibroblasts, suggesting that the dynamic culture protocol could be system-specific and may require optimization for different tissue types. While convection was introduced to the hChaMP via rocking, due to the complexity of the hChaMP, a perfusion bioreactor may be beneficial to ensure adequate flow through the chambers and to mimic physiological conditions more precisely. Perfusion bioreactors have been shown to be capable of recapitulating pulsatile flow profiles and combined with mechanical and electrical stimulation to simulate the heart physiology environment, enable recellularized cardiac ECM constructs to achieve cell density similar of native myocardium, and perform stroke work. Given this consideration, together with the fact that cardiac biomechanics are continually changing not only during the contraction cycle but also by developmental stage, a dynamic culture protocol with time-variant properties over both long and short time scales conducted by a perfusion bioreactor may be used for further improving tissue robustness and physiological functions of the hChaMP.

In addition to manipulating the external culture system, another approach for increasing tissue thickness may be to maintain nutrient and oxygen supply within the engineered tissue by means of a well-developed vascular network. Incorporation of vascular endothelial cells and fibroblasts in cardiac patches has been shown to significantly increase vessel lumen formation and thus cell viability after transplantation. Combined iPSC-derived cardiomyocytes and vascular cells may enhanced tissue maturation *in vitro* and generated vascular structures as well as functional implant coupling *in vivo.* In addition, co-culture of endothelial cells with cardiomyocytes may result in tubular lumens which can form a perfusable network with an external vascular bed. Inclusion of pre-vascularized structures during tissue formation could be another approach to achieve functional vasculature. 3D printing may be particularly well suited to attaining this goal because it allows for spatial control of both materials and cells. Vascular structures can be printed with either endothelial cell-laden bioink or sacrificial materials followed by endothelialization, resulting in functional vascularization throughout the printed constructs. As described herein, endothelial differentiation and tube formation were not intentionally included, but future design iterations should include stimulants of endothelial differentiation and vascular network formation. As one possible approach, a 3D printed microcapsule-based system could be a powerful means to vascularize hChaMPs by taking advantage of spatially-defined stem cell differentiation.

In addition to enhancing the thickness of the cardiac muscle within the hChaMP, improvement of this model may include enhanced maturation of cardiomyocytes and corresponding pump function. The expression of maturation markers are associated with cell-cell junctions, ion handling, and excitation-contraction coupling, but mature cell alignment and sarcomeric organization are still lacking. Furthermore, in addition to structural organization, multiple metrics of functional maturation should also be met to augment the performance of a living pump.

The perpetual criticism of stem cell-derived cardiomyocytes is that they are not structurally or functionally mature and thus a large body of literature is emerging to address this deficiency. Most popular is the imposition of electrical and mechanical stimulation on stem cell-derived cardiomyocytes in 2D culture or in engineered tissues. Of note, field stimulation of stem cell-derived cardiomyocytes in a 3D tissue has been shown to promote improvements in ion handling and action potential propagation, as well as cell alignment and structural maturation. Furthermore, by combining field stimulation with mechanical loading, researchers have generated 3D tissues with neonatal rat cardiomyocytes that demonstrate a positive force-frequency response, a critical characteristic of native cardiac tissue. In another case, a controlled afterload was imposed on hiPSC-derived cardiac tissues, which subsequently exhibited a positive Frank-Starling relationship.

Hence, it seems likely that introducing electro-mechanical conditioning to the hChaMP could significantly improve maturation and resulting function. In addition, with some modifications and improvements, the hChaMP provides a unique system to investigate the characteristic Frank-Starling relationship of the native heart. While *in vitro* assessment of this relationship in most tissue-engineered constructs is based on length versus resulting force of contraction, physiologically it is a metric that relates stroke volume to the end-diastolic volume. A tissue engineered construct that can hold volume is therefore a benefit to recapitulate this relationship. The main limitation to achieving this with the hChaMP is the lack of valvular structures, which precludes the generation of a controlled pre-load. A perfusion bioreactor that incorporates valves would allow for pressure build-up within the hChaMP as well as control over fill volume of the construct. Synchronized with field stimulation, such a system would allow for electromechanical conditioning to promote maturation, generation of more physiological pressure-volume loops, and the capacity to measure the Frank-Starling relationship based on stroke volume and preload. In the short term, the most feasible avenue to achieve this end would be the incorporation of mechanical valves into tubing that attaches to the vessel inlet and outlets. However, tissue engineered valves may also be employed, which would enable the generation of four-chambered structures with valves for both atrial and ventricular filling.

In incorporating electro-mechanical stimulation into the hChaMP, a factor to consider will be timing of conditioning. The stage at which stem cell derived cardiomyocytes are mechanically stimulated after differentiation may have an impact on the level of the resulting maturation and that early-stage cardiomyocytes are more responsive to this treatment. However, because cardiomyocytes of the hChaMP are differentiated *in situ,* conditioning could hypothetically be imposed even earlier than this, prior to completion of differentiation. Electrical stimulation of progenitor cells may be derived from epicardial fat enhances maturation of the cardiomyocytes that differentiate from these cells. This suggests that electromechanical stimulation mid-differentiation could be beneficial in the context of the hChaMP.

In addition to electrical and mechanical stimuli, there are a myriad of soluble factors that have been explored to promote cardiomyocyte maturation. Among these are the hormone tri-iodo-L-thyronine, the alpha-adrenergic agonist phenylephrine, and insulin-like growth factor. MicroRNAs have also been shown to play a key role in driving metabolic maturation of stem cell derived cardiomyocytes. Incorporation of soluble signals along with electrical and physical cues could provide an avenue to further mature cardiomyocytes of the hChaMP.

Therefore, the bioink and structures described herein can enable macroscale beating function in a complex, chambered structure. This outcome was made possible by an example bioink that allowed extensive stem cell proliferation prior to differentiation to yield contiguous muscle walls of up to 500 µm in thickness. This approach could be applied to many other cell types with poor proliferative and migratory capacity following differentiation. In the end, the living human pump shown here and future design iterations will find utility for multiscale *in vitro* cardiology assays, injury and disease modeling, medical device testing, and regenerative medicine research that should more easily transfer to clinically relevant outcomes.

The following examples are described herein. Example 1: a bioink composition comprising gelatin methacrylate and collagen methacrylate.

Example 2: the bioink composition of example 1, further comprising lithium phenyl-2,4,6-trimethylbenzoylphosphinate.

Example 3: The bioink composition of example 2, further comprising a solvent comprising: mTeSR medium; acetic acid; and sodium hydroxide (NaOH).

Example 4: The bioink composition of example 3, wherein the solvent comprises approximately: 74 percent weight by volume of mTeSR medium; 20 percent weight by volume of 20mM acetic acid; and 1 percent weight by volume of 1M NaOH.

Example 5: the bioink composition of any of examples 1 through 4, wherein the bioink composition comprises: approximately 10 percent weight by volume of the gelatin methacrylate; approximately 0.25 percent weight by volume of the collagen methacrylate; and approximately 0.5 percent weight by volume of the lithium phenyl-2,4,6-trimethylbenzoylphosphinate.

Example 6: the bioink composition of any of examples 1 through 5, further comprising at least one of fibronectin or laminin.

Example 7: the bioink composition of any of examples 1 through 6, wherein the composition comprises: approximately 100 milligrams per milliliter (mg/mL) of the collagen methacrylate; approximately 2.5 mg/mL of the gelatin methacrylate; approximately 5 mg/mL of the of the lithium phenyl-2,4,6-trimethylbenzoylphosphinate; approximately 93.8 micrograms per milliliter (µg/mL) of the fibronectin; and approximately 93.8 µg/mL of the laminin.

Example 8: the bioink composition of any of examples 1 through 7, further comprising human induced pluripotent stem cells.

Example 9: the bioink composition of example 8, wherein the human induced pluripotent stem cells comprise human induced pluripotent stem cells overexpressing cyclin D2 (CCND2).

Example 10: the bioink composition of example 8, wherein the human induced pluripotent stem cells comprise cardiomyocyte precursors.

Example 11: the bioink composition of any of examples 1 through 10, further comprising cardiomyocytes.

Example 12: the bioink composition of any of examples 1 through 11, wherein the bioink composition is configured to promote differentiation of human induced pluripotent stem cells into cardiomyocytes.

Example 13: a method comprising printing a three-dimensional structure using the bioink composition of any of examples 1 through 12.

Example 14: the method of example 13, wherein printing the three-dimensional structure using the bioink composition of any of claims 1 through 12 comprises printing the three-dimensional structure using the bioink to create at least one chamber.

Example 15: the method of any of examples 13 through 14, wherein the three-dimensional structure comprises human induced pluripotent stem cells, and wherein the method further comprises inducing the human induced pluripotent stem cells to differentiate into cardiomyocytes by modulating a Wnt/β-catenin pathway with small molecules.

Example 16: a method comprising printing a three-dimensional structure using a bioink comprised of gelatin methacrylate, collagen methacrylate, and lithium phenyl-2,4,6-trimethylbenzoylphosphinate.

Example 17: the method of example 16, wherein printing the three-dimensional structure using the bioink comprises printing the three-dimensional structure using the bioink to create at least one chamber.

Example 18: the method of any of examples 16 and 17, wherein the three-dimensional structure comprises human induced pluripotent stem cells, and wherein the method further comprises inducing the human induced pluripotent stem cells to differentiate into cardiomyocytes by modulating a Wnt/β-catenin pathway with small molecules.

Example 19: the method of any of examples 16 through 18, wherein inducing the human induced pluripotent stem cells to differentiate into cardiomyocytes comprises inducing the human induced pluripotent stem cells to differentiate into cardiomyocytes having a cell density approximating cardiac tissue.

Example 20: a bioink composition comprising: gelatin methacrylate; collagen methacrylate; lithium phenyl-2,4,6-trimethylbenzoylphosphinate; fibronectin; laminin; and a solvent comprising mTeSR medium, acetic acid, and sodium hydroxide (NaOH).

Example 21: the bioink composition of example 21, wherein the bioink composition comprises: approximately 10 percent weight by volume of the gelatin methacrylate; approximately 0.25 percent weight by volume of the collagen methacrylate; and approximately 0.5 percent weight by volume of the lithium phenyl-2,4,6-trimethylbenzoylphosphinate.

Various examples have been described. These and other examples are within the scope of the following claims.

## Claims

1. A bioink composition comprising:
5 to 20 percent weight by volume of gelatin methacrylate; and
0.1 to 1.0 percent weight by volume of collagen methacrylate

2. The bioink composition of claim 1, further comprising lithium phenyl-2,4,6-trimethylbenzoylphosphinate.

3. The bioink composition of claim 2, further comprising a solvent comprising:
mTeSR medium;
acetic acid; and
sodium hydroxide (NaOH).

4. The bioink composition of claim 3, wherein the solvent comprises 74 percent weight by volume of mTeSR medium;
20 percent weight by volume of 20mM acetic acid; and
1 percent weight by volume of 1M NaOH.

5. The bioink composition of any of claims 1 through 4, wherein the bioink composition comprises:
10 percent weight by volume of the gelatin methacrylate;
0.25 percent weight by volume of the collagen methacrylate; and
0.5 percent weight by volume of the lithium phenyl-2,4,6-trimethylbenzoylphosphinate.

6. The bioink composition of any of claims 1 through 5, further comprising at least one of fibronectin or laminin.

7. The bioink composition of any of claims 1 through 6, wherein the composition comprises:
100 milligrams per milliliter (mg/mL) of the collagen methacrylate;
2.5 mg/mL of the gelatin methacrylate;
5 mg/mL of the of the lithium phenyl-2,4,6-trimethylbenzoylphosphinate;
93.8 micrograms per milliliter (µg/mL) of the fibronectin; and
93.8 µg/mL of the laminin.

8. The bioink composition of any of claims 1 through 7, further comprising human induced pluripotent stem cells.

9. The bioink composition of claim 8, wherein the human induced pluripotent stem cells comprise human cardiac fibroblast-derived induced pluripotent stem cells overexpressing Cyclin D2 (CCND2) under the myosin heavy chain (MHC).

10. The bioink composition of claim 8, wherein the human induced pluripotent stem cells comprise cardiomyocyte precursors.

11. The bioink composition of any of claims 1 through 10, further comprising cardiomyocytes.

12. The bioink composition of any of claims 1 through 11, wherein the bioink composition is configured to promote differentiation of human induced pluripotent stem cells into cardiomyocytes.

13. A method comprising:
printing a three-dimensional structure using the bioink composition of any of claims 1 through 12.

14. The method of claim 13, wherein printing the three-dimensional structure using the bioink composition of any of claims 1 through 12 comprises printing the three-dimensional structure using the bioink to create at least one chamber.

15. The method of any of claims 13 through 14, wherein the three-dimensional structure comprises human induced pluripotent stem cells, and wherein the method further comprises inducing the human induced pluripotent stem cells to differentiate into cardiomyocytes by modulating a Wnt/β-catenin pathway with small molecules.

## Patentansprüche

1. Eine Biotintenzusammensetzung, die Folgendes beinhaltet:
5 bis 20 Gewichts-/Volumenprozent an Gelatinemethacrylat; und
0,1 bis 1,0 Gewichts-/Volumenprozent an Kollagenmethacrylat.

2. Biotintenzusammensetzung gemäß Anspruch 1, ferner beinhaltend Lithium-Phenyl-2,4,6-trimethylbenzoylphosphinat.

3. Biotintenzusammensetzung gemäß Anspruch 2, ferner beinhaltend ein Lösemittel, beinhaltend:
mTeSR-Medium;
Essigsäure; und
Natriumhydroxid (NaOH).

4. Biotintenzusammensetzung gemäß Anspruch 3, wobei das Lösemittel beinhaltet 74 Gewichts-/Volumenprozent an mTeSR-Medium;
20 Gewichts-/Volumenprozent an 20 mM Essigsäure; und
1 Gewichts-/Volumenprozent an 1 M NaOH.

5. Biotintenzusammensetzung gemäß einem der Ansprüche 1 bis 4, wobei die Biotintenzusammensetzung Folgendes beinhaltet:
10 Gewichts-/Volumenprozent des Gelatinemethacrylats:
0,25 Gewichts-/Volumenprozent des Kollagenmethacrylats; und
0,5 Gewichts-/Volumenprozent des Lithium-Phenyl-2,4,6-trimethylbenzoylphosphinats.

6. Biotintenzusammensetzung gemäß einem der Ansprüche 1 bis 5, ferner beinhaltend mindestens eines von Fibronectin oder Laminin.

7. Biotintenzusammensetzung gemäß einem der Ansprüche 1 bis 6, wobei die Zusammensetzung Folgendes beinhaltet:
100 Milligramm pro Milliliter (mg/ml) des Kollagenmethacrylats:
2,5 mg/ml des Gelatinemethacrylats;
5 mg/ml des Lithium-Phenyl-2,4,6-trimethylbenzoylphosphinats;
93,8 Mikrogramm pro Milliliter (µg/ml) des Fibronectins; und
93,8 µg/ml des Laminins.

8. Biotintenzusammensetzung gemäß einem der Ansprüche 1 bis 7, ferner beinhaltend induziert pluripotente menschliche Stammzellen.

9. Biotintenzusammensetzung gemäß Anspruch 8, wobei die induziert pluripotenten menschlichen Stammzellen aus Herzfibroblasten gewonnene induziert pluripotente menschliche Stammzellen beinhalten, die Cyclin D2 (CCND2) unter der schweren Myosin-Kette (MHC) überexprimieren.

10. Biotintenzusammensetzung gemäß Anspruch 8, wobei die induziert pluripotenten menschlichen Stammzellen Kardiomyocyten-Vorläufer beinhalten.

11. Biotintenzusammensetzung gemäß einem der Ansprüche 1 bis 10, ferner beinhaltend Kardiomyocyten.

12. Biotintenzusammensetzung gemäß einem der Ansprüche 1 bis 11, wobei die Biotintenzusammensetzung konfiguriert ist, um Differenzierung von induziert pluripotenten menschlichen Stammzellen zu Kardiomyocyten zu fördern.

13. Ein Verfahren, beinhaltend:
Drucken einer dreidimensionalen Struktur unter Verwendung der Biotintenzusammensetzung gemäß einem der Ansprüche 1 bis 12.

14. Verfahren gemäß Anspruch 13, wobei das Drucken der dreidimensionalen Struktur unter Verwendung der Biotintenzusammensetzung gemäß einem der Ansprüche 1 bis 12 das Drucken der dreidimensionalen Struktur unter Verwendung der Biotinte beinhaltet, um mindestens eine Kammer zu erzeugen.

15. Verfahren gemäß einem der Ansprüche 13 bis 14, wobei die dreidimensionale Struktur induziert pluripotente menschliche Stammzellen beinhaltet, und wobei das Verfahren ferner das Induzieren der induziert pluripotenten menschlichen Stammzellen beinhaltet, um zu Kardiomyocyten zu differenzieren, indem ein Wnt/β-Catenin-Signalweg mit kleinen Molekülen moduliert wird.

## Revendications

1. Une composition de bio-encre comprenant :
de 5 à 20 pour cent en poids par volume de méthacrylate de gélatine ; et
de 0,1 à 1,0 pour cent en poids par volume de méthacrylate de collagène.

2. La composition de bio-encre de la revendication 1, comprenant en outre du phényl-2,4,6-triméthylbenzoylphosphinate de lithium.

3. La composition de bio-encre de la revendication 2, comprenant en outre un solvant comprenant :
du milieu mTeSR ;
de l'acide acétique ; et
de l'hydroxyde de sodium (NaOH).

4. La composition de bio-encre de la revendication 3, où le solvant comprend 74 pour cent en poids par volume de milieu mTeSR ;
20 pour cent en poids par volume d'acide acétique à 20 mM ; et
1 pour cent en poids par volume de NaOH à 1 M.

5. La composition de bio-encre de n'importe lesquelles des revendications 1 à 4, la composition de bio-encre comprenant :
10 pour cent en poids par volume du méthacrylate de gélatine ;
0,25 pour cent en poids par volume du méthacrylate de collagène ; et
0,5 pour cent en poids par volume du phényl-2,4,6-triméthylbenzoylphosphinate de lithium.

6. La composition de bio-encre de n'importe lesquelles des revendications 1 à 5, comprenant en outre de la fibronectine et/ou de la laminine.

7. La composition de bio-encre de n'importe lesquelles des revendications 1 à 6, la composition comprenant :
100 milligrammes par millilitre (mg/mL) du méthacrylate de collagène ;
2,5 mg/mL du méthacrylate de gélatine ;
5 mg/mL du phényl-2,4,6-triméthylbenzoylphosphinate de lithium ;
93,8 microgrammes par millilitre (µg/mL) de la fibronectine ; et
93,8 µg/mL de la laminine.

8. La composition de bio-encre de n'importe lesquelles des revendications 1 à 7, comprenant en outre des cellules souches pluripotentes induites humaines.

9. La composition de bio-encre de la revendication 8, où les cellules souches pluripotentes induites humaines comprennent des cellules souches pluripotentes induites dérivées de fibroblastes cardiaques humains surexprimant la Cycline D2 (CCND2) sous la chaîne lourde de la myosine (MHC).

10. La composition de bio-encre de la revendication 8, où les cellules souches pluripotentes induites humaines comprennent des précurseurs de cardiomyocyte.

11. La composition de bio-encre de n'importe lesquelles des revendications 1 à 10, comprenant en outre des cardiomyocytes.

12. La composition de bio-encre de n'importe lesquelles des revendications 1 à 11, la composition de bio-encre étant configurée pour promouvoir la différenciation de cellules souches pluripotentes induites humaines en cardiomyocytes.

13. Un procédé comprenant :
l'impression d'une structure tridimensionnelle à l'aide de la composition de bio-encre de n'importe lesquelles des revendications 1 à 12.

14. Le procédé de la revendication 13, où l'impression de la structure tridimensionnelle à l'aide de la composition de bio-encre de n'importe lesquelles des revendications 1 à 12 comprend l'impression de la structure tridimensionnelle à l'aide de la bio-encre afin de créer au moins une chambre.

15. Le procédé de n'importe lesquelles des revendications 13 à 14, où la structure tridimensionnelle comprend des cellules souches pluripotentes induites humaines, et le procédé comprenant en outre l'induction des cellules souches pluripotentes induites humaines afin qu'elles se différencient en cardiomyocytes en modulant une voie Wnt/β-caténine avec de petites molécules.
